# EUROPÄISCHE PATENTSCHRIFT

(11) **EP 3 908 131 B1**
(45) Veröffentlichungstag und Bekanntmachung des Hinweises auf die Patenterteilung: **17.04.2024**
(21) Anmeldenummer: 19827605.7
(22) Anmeldetag: 10.12.2019
(51) Int. Cl.: A61K 31/05, A61K 31/352, A61M 11/00, A61M 11/02, A61M 11/04, A61M 15/06, A24F 40/42, A61M 15/00, A61J 3/00, A61K 36/185, A61M 11/06, A61M 16/00, G01N 33/94

(54) **VORRICHTUNG UND VERFAHREN ZUM EXTRAHIEREN UND ASPIRIEREN VON WIRKSTOFFEN, INSBESONDERE AUS DER CANNABISPFLANZE**
DEVICE AND METHOD FOR THE EXTRACTION AND ASPIRATION OF ACTIVE SUBSTANCES, IN PARTICULAR FROM THE CANNABIS PLANT
DISPOSITIF ET PROCÉDÉ POUR L'EXTRACTION ET L'ASPIRATION DE SUBSTANCES ACTIVES, EN PARTICULIER DE CANNABIS

(30) Priorität: 07.01.2019 DE 102019000018; 07.01.2019 DE 102019000016; 15.01.2019 DE 102019000199
(43) Veröffentlichungstag der Anmeldung: 17.11.2021
(73) Patentinhaber: LuxCan Innovation S.A., 1273 Luxembourg-Hamm (LU); Schmitt, Fritz, 1855 Luxemburg (LU)
(72) Erfinder: SCHMITT, Fritz, 1855 Luxemburg (LU)
(74) Vertreter: Angerhausen, Christoph
(86) Internationale Anmeldenummer: PCT/DE2019/101068
(87) Internationale Veröffentlichungsnummer: WO 2020/143865

(56) Entgegenhaltungen:
- WO-A1-2014/150826
- WO-A1-2017/108987
- WO-A1-2017/207674
- WO-A1-2019/105811
- CN-U- 205 648 930
- US-A1- 2017 055 574
- US-A1- 2017 150 753
- US-A1- 2018 027 883

## Beschreibung

Die Erfindung betrifft eine Kapsel zur Verwendung in einem Verdampfer, mit mindestens einem zumindest teilweise von einem Deckel, Seitenwänden und Boden der Kapsel gebildeten ersten Hohlraum und einem in dem ersten Hohlraum der Kapsel aufgenommenen, verdampfbaren Wirkstoff. Die Erfindung betrifft weiter eine Anordnung aus einer solchen Kapsel und einem Verdampfer, vorzugsweise einen handtragbaren Verdampfer, sowie ein System aus Verdampfer und in dem Verdampfer aufgenommener Kapsel. Es werden insbesondere auch Vorrichtungen und Verfahren zum Extrahieren und Aspirieren von Wirkstoffen aus der Cannabispflanze beschrieben. Eine Kapsel gemäß dem Oberbegriff des Anspruch 1 ist aus der US 2018/027883 A1 bekannt. Ähnliche Vorrichtungen beschreiben auch die WO 2017/108987 A1, die US 2017/055574 A1, die WO 2019/105811 A1, die US 2017/150753 A1, die WO 2014/150826 A1, die WO 2017/207674 A1 und die CN 205648930 U.Verdampfer bzw. Vaporizer kommen seit vielen Jahrzehnten in der Medizin und in der Naturheilkunde zum Einsatz, besonders bei Erkrankungen der Lunge und der Atemwege sowie in der Schmerztherapie. Da die Wirkstoffe über die Lunge aufgenommen werden, ist eine Wirkung relativ schnell spürbar.

In der klassischen Aromatherapie werden ätherische Öle aus Pflanzenextrakten bzw. Heilkräutern wie Kamille, Sandelholz oder Rosmarin verdampft. Hingegen werden z.B. in der Phytotherapie Pflanzenextrakte verdampft, wobei jeder Wirkstoff einen anderen Siedepunkt besitzt und dementsprechend eine bestimmte Temperatur benötigt, um die optimale Wirkung entfalten zu können. Im Vergleich zum Verdampfen von ätherischen Ölen ist das direkte Verdampfen von Kräutern sparsamer und effizienter.

Wenn Cannabis für medizinische Zwecke genutzt wird, sollten sich möglichst viele Cannabinoide wie THC und CBD aus der Cannabispflanze lösen. Jedoch liegen die Cannabinoide in der Cannabispflanze nicht ihrer Reinform, sondern als Carboxylsäuren wie THCA und CBDA vor, die zu THC und CBD umgewandelt werden müssen. Erreicht wird das durch den Decarboxylierungsprozess, indem mithilfe von Hitze von den Carboxylsäuren jeweils ein Molekül Kohlendioxid abgespaltet wird und die Verbindungen THC und CBD übrig bleiben. Die dafür notwendige Wärme kann durch Verdampfung oder Verbrennung des Wirkstoffs bzw. der Cannabispflanze oder Kräuter bereitgestellt werden.

Unter Verbrennung wird üblicherweise eine Freisetzung chemisch gebundener Energie in Wärme durch Stoffumwandlung verstanden. Dabei werden Brennstoff und Sauerstoff als Ausgangsstoffe der Reaktion, sogenannte Edukte, in Endprodukte wie Kohlendioxid und Wasser in einer Folge von chemischen Reaktionen umgesetzt. Während des Ablaufs dieser Reaktionen, die auch als Kettenreaktionen bezeichnet werden, werden Zwischenprodukte wie Kohlenmonoxid gebildet; es können darüber hinaus aber auch Schadstoffe wie Stickstoffmonoxid und Ruß auftreten. Die Reaktionsgeschwindigkeit der Elementarreaktionen, insbesondere die die Verbrennung aufrecht erhaltenden Kettenverzweigungsreaktionen, sind dabei unter anderem temperaturabhängig, d.h. erst bei Überschreiten einer unter anderem von dem Brennstoff abhängigen Temperatur kann eine stabile Verbrennung erfolgen.

Bei der Verbrennung bzw. beim Rauchen von Cannabis entsteht in der Glut eine Hitze zwischen 800 und 900 °C, die zur Umwandlung der Carboxylsäure THCA in THC ausreichend ist. Allerdings enthalten ungefähr 88% der bei der Verbrennung erzeugten Stoffe keine Cannabinoide; ein Größteil der Cannabinoide wird durch die Verbrennung bzw. die hohe Temperatur zerstört. Zudem werden durch die Verbrennung von Cannabis mit einer offenen Flamme und hohen Temperaturen schädliche Chemikalien freigesetzt, beispielsweise Teer, Benzol oder Kohlenmonoxid.

Bei dem Verdampfen von Cannabis ist hingegen eine Temperatur von über 185 Grad Celsius anzustreben. Die Carboxylsäuren und Terpene werden bei einer Temperatur von 210 Grad Celsius nahezu vollständig herausgelöst, ohne dass das Pflanzenmaterial verbrennt. Beim Verdampfen von Cannabis werden zudem nicht die bei der Verbrennung freigesetzten toxischen Stoffe erzeugt, da das Cannabis nur bis zu dem Punkt erhitzt wird, ab welchem sich die Cannabinoide zu Gas umwandeln. Dieser Punkt liegt üblicherweise bei ca. 200°C.

Das Verdampfen des Wirkstoffs, z.B. einer Cannabisblüte, bietet weitere entscheidende Vorteile. So kann eine große Menge der Inhaltsstoffe beim Inhalieren aufgenommen werden; der Wirkungseintritt findet schon nach ein bis zwei Minuten statt und kann zwei bis vier Stunden anhalten. Untersuchungen zeigen, dass beim Inhalieren bis zu einem Drittel der Cannabinoide aus dem Cannabis ins Blut aufgenommen werden, während z.B. bei einer oralen Einnahme von Cannabis-Medikamenten oder Cannabisextrakten nur ein Neuntel aufgenommen wird. Zudem ergibt sich ein schneller Wirkeintritt innerhalb von wenigen Minuten, während bei einer oralen Einnahme von Cannabis-Medikamenten oder Cannabisextrakten bis zu 90 Minuten zum Eintritt der Wirkung vergehen können. Da die Aufnahme der Wirkstoffe in den Blutkreislauf rasch erfolgt, kann die richtige Dosierung leicht gefunden werden. Dies ist besonders bei den ersten Anwendungen hilfreich. Ein Patient kann sich z.B. langsam an die vom Arzt empfohlene Dosierung herantasten. Weiterhin ist der durch den Verdampfer entstandene Dampfgeruch sehr mild und nur von kurzer Dauer.

Ein bekannter handtragbarer Verdampfer wird von dem Unternehmen Storz & Bickel unter dem Namen "Mighty Medic" vertrieben. Der Benutzer kann Kräuter, insbesondere gemahlene Cannabisblätter oder Hanfblüten, in mehrfach verwendbare Kapseln füllen und dabei den Füllstand selbst dosieren. Die Kapsel weist eine einfache Wand auf; der Deckel ist als Sieb ausgebildet. Vor der Benutzung im Verdampfer müssen die manuell gefüllten Kapseln in einem separaten Kapselmagazin gelagert werden, um die Kapseln bzw. die in den Kapseln aufgenommenen gemahlenen Cannabisblätter oder Hanfblüten vor Umwelteinflüssen und Verunreinigungen zu schützen.

Damit ergibt sich zum einen eine umständliche Lagerung der befüllten Kapseln; die Wirkstoffe im Kapselinneren können in die Umgebung diffundieren oder sich verflüchtigen, so dass die Haltbarkeit der Wirkstoffe eingeschränkt wird . Zum anderen kann bei Einlagerung oder Entnahme der Kapseln aus dem Kapselmagazin Keime, Verunreinigungen, Staub, oder dergleichen in die Kapseln eintreten, selbst wenn das Kapselmagazin selbst frei von Keimen, Verunreinigungen oder dergleichen sein sollte. Auch durch die Merhfachverwendung der Kapsel können Keime oder dergleichen in das Kapselinnere gelangen. Zudem können durch die manuelle Befüllung der Kapsel Fehldosierungen auftreten oder der Füllstand variieren. Insbesondere wenn verschiedene Wirkstoffe in der Kapsel verdampft werden sollen, oder mehrere Stoffe in einer bestimmten Art zusammenwirken sollen, ist eine manuelle Befüllung nachteilig, da nicht nur die jeweilige Menge sondern auch die Vermischung relevant sein können.

Es ist deshalb die Aufgabe der vorliegenden Erfindung, eine Kapsel bereitzustellen, die eine erhöhte Hygiene und eine verbesserte Kapselfunktionalität bereitstellt.

Diese Aufgabe wird durch eine Kapsel mit den Merkmalen des Anspruchs 1, eine Anordnung mit den Merkmalen des Anspruchs 12 sowie ein Verdampfersystem nach Anspruch 17 gelöst. Vorteilhafte Ausführungsformen sind Gegenstand der Unteransprüche.

Demnach weist der Boden der Kapsel zumindest an seiner Außenseite zumindest teilweise ein Material, bevorzugt ein Metall, mit einer verglichen mit dem Material der Seitenwand höheren Wärmeleitfähigkeit auf..

Der weitere Wirkstoff kann von dem in dem ersten Hohlraum der Kapsel aufgenommenen Wirkstoff unterschiedlich oder gleichartig sein. Es kann vorgesehen sein, dass der weitere Wirkstoff die Wirkung des in dem ersten Hohlraum aufgenommenen Wirkstoffs verstärken oder mit diesem zusammenwirken kann. Das chemische Reaktionsmittel kann dazu vorgesehen sein, durch chemische Reaktion Wärme freizusetzen. Der Hilfsstoff kann verdampfbar sein.

Der Wirkstoff kann ein pflanzlicher Wirkstoff sein oder aufweisen. Das chemische Reaktionsmittel kann beispielsweise ein Säureanhydrid oder ein saures Salz und ein basisches Anhydrid oder ein basisches Salz sein oder aufweisen, oder eine Mischung aus diesen. Das basische Salz kann aus der Gruppe gewählt sein, welche aus Natriumacetat, Natriumbenzoat und Kaliumascorbat besteht oder diese umfasst. Das Geschmacksmittel kann z.B. ein Aromastoff, ein Geschmacksverstärker, ein Duftstoff oder dergleichen sein oder aufweisen.

Wird nachfolgend auf den Wirkstoff Bezug genommen, kann auch der Wirkstoff und/oder der Hilfsstoff gemeint sein.

Insbesondere kann vorgesehen sein, die Kapsel als Ein-Wege-Kapsel auszuführen. Damit kann die Zusammensetzung des Wirkstoffs in der ersten Hohlkammer sowie des in der zweiten Hohlkammer aufgenommenen Hilfsstoffs bei der Kapselproduktion vorgegeben sein. Damit kann die Handhabbarkeit der Kapsel durch den Benutzer erleichtert werden. Es kann vorgesehen sein, für bestimmte Anwendungsbereiche der Kapsel verschiedene Konzentrationen und/oder Kombinationen der jeweiligen Wirkstoffe vorzusehen.

Es können auch mehrere separate Hohlräume zwischen der Innenwand und der Außenwand ausgebildet sein. Es kann auch vorgesehen sein, den ersten Hohlraum in mehrere, ggf. separate bzw. voneinander getrennte und/oder abgeschlossene Unterhohlräume aufzuteilen oder zu partitionieren. Der Begriff "doppelwandig" kann auch mindestens abschnittsweise mehrwandige Seitenwände und/oder Boden umfassen. Beispielsweise kann eine doppelwandige Seitenwand eine dem ersten Hohlraum zugewandte Innenwand, eine der Außenseite der Kapsel zugewandte Außenwand sowie mindestens abschnittsweise eine oder mehrere zwischen Innenwand und Außenwand angeordneten Zwischenwände aufweisen. Dabei können mehrere Zwischenhohlräume ausgebildet sein.

Die Seitenwände, die Innenwand, die Außenwand, der Deckel und/oder der Boden der Kapsel kann aus einem Kunststoff, einem Keramik, einem Glas oder einem Metall bestehen oder aufweisen. Der Deckel der Kapsel kann eine Folie sein oder aufweisen. Damit kann von der Produktion der Kapseln bis hin zur Verwendung im Verdampfer ein hygienischer Wirkstoff im Kapselinneren und eine genaue Dosierung sichergestellt werden.

Die Außenwand, der Deckel und der Boden können luftundurchlässig sein, so dass der erste und/oder der zweite Hohlraum hermetisch gegen eine Umgebung der Kapsel abgeschlossen sein kann. Es kann vorgesehen sein, dass der Deckel mit den Seitenwänden versiegelt ist. Da die Hohlräume hermetisch gegen die Umgebung abgeschlossen sind, kann der Wirkstoff und/oder der Hilfsstoff fest, flüssig und/oder gasförmig in der Kapsel vorliegen. Zudem werden Verunreinigungen des in die Kapsel aufgenommenen Wirkstoffs verhindert oder zumindest deutlich verringert, die Handhabung und Lagerung erleichtert und eine längere Haltbarkeit des Wirkstoffs erreicht. Insbesondere kann die Kapsel vorteilhaft als Ein-Wege-Kapsel ausgeführt sein.

Der der erste und/ oder der zweite Hohlraum kann entweder evakuiert oder zumindest teilweise mit einem Schutzgas befüllt sein. Durch die Evakuierung bzw. das Vakuum oder die Schutzgasatmosphäre im Hohlraum kann die Haltbarkeit des Wirkstoffs erhöht werden. Insbesondere kann die Kapsel über einen langfristigen Zeitraum gelagert werden, ohne dass die Wirksamkeit des in der Kapsel aufgenommenen Wirkstoffs beeinträchtigt wird. Zudem kann damit die Verdampfung erleichtert und/oder die Verbrennung in Ermangelung von Sauerstoff verhindert werden. Alternativ oder zusätzlich kann auch vorgesehen sein, dass der erste und/oder der zweite Hohlraum mit Druckluft oder einem Treibgas befüllt ist.

Die zwischen dem ersten und zweiten Hohlraum angeordnete Innenwand der Seitenwand kann perforierbar, porös und/oder semi-permeabel sein und/oder Sollbruchstellen aufweisen. Dadurch kann vorgesehen sein, eine Verbindung beziehungsweise eine Öffnung zwischen erstem und zweitem Hohlraum gezielt oder kontrolliert und/oder zu einem bestimmten Zeitpunkt und/oder unter bestimmten Bedingungen herzustellen. In einigen Ausführungsformen kann aber auch vorgesehen sein, dass der erste und der zweite Hohlraum beim Verdampfen des Wirkstoffs und des Hilfsstoffs voneinander fluidisch getrennt bleiben, d.h. der Wirkstoff und der Hilfsstoff separat verdampft werden. Es kann vorgesehen sein, die verdampften Stoffe erst nach der jeweiligen Verdampfung zu vermischen. In einigen Ausführungsformen kann die Verdampfung des Wirkstoffs und des Hilfsstoffs auch zeitlich getrennt, nacheinander oder zumindest zeitlich überlappend stattfinden.

Der Deckel kann perforierbar sein und/oder Sollbruchstellen und/oder Ventile aufweisen. Dadurch kann eine Öffnung in dem Deckel erzeugt werden, so dass der in dem ersten Hohlraum aufgenommene Wirkstoff und/oder der in dem zweiten Hohlraum aufgenommene Hilfsstoff mit der Umgebung der Kapsel interagieren kann bzw. aus der Kapsel austreten kann.

Wird der in dem ersten Hohlraum aufgenommene Wirkstoff beispielsweise verdampft, so kann der verdampfte Wirkstoff aus der Kapsel durch die Öffnung im Deckel austreten. Ist der zweite Hohlraum durch die Innenwand zum ersten Hohlraum geöffnet, so kann der verdampfte Hilfsstoff in den ersten Hohlraum eintreten und durch den Deckel aus dem ersten Hohlraum austreten. Alternativ oder zusätzlich kann der Deckel aber auch im Bereich des zweiten Hohlraums geöffnet werden, so dass der Hilfsstoff aus dem zweiten Hohlraum durch den Deckel austreten kann.

Der Wirkstoff kann eine Cannabisblüte, ein Cannabisöl, ein Cannabinoid, ein Cannabisextrakt oder dergleichen sein oder aufweisen. Der Wirkstoff kann flüssig, fest oder gasförmig sein. Bevorzugt ist der Wirkstoff eine Cannabispflanze oder ein oder mehrere Cannabisblätter oder dergleichen. Die in der Kapsel aufgenommene Cannabispflanze bzw. die Cannabisblätter können befeuchtet sein. Ein Verdampfer, in den die Kapsel einsetzbar ist, kann bspw. einen Mikrowellengenerator aufweisen, so dass die befeuchtete Cannabispflanze bzw. die befeuchteten Cannabisblätter z.B. auf Temperaturen zwischen 180 - 220°C erwärmt werden können und der Wirkstoff verdampft werden kann.

Ist der Boden doppelwandig, so kann vorgesehen sein, dass ein wärmeleitender Stoff zwischen der Außenseite und der dem ersten Hohlraum der Kapsel zugewandten Innenseite des Bodens aufgenommen ist. Damit kann eine gute Wärmeübertragung zwischen der Außenseite des Bodens und der Innenseite bereitgestellt sein.

Der Deckel und/oder der Boden kann an einer dem Hohlraum zugewandten Innenseite mindestens teilweise eine Beschichtung aufweisen. Alternativ oder zusätzlich kann die Innenwand mindestens teilweise eine Beschichtung aufweisen. Die Beschichtung kann bevorzugt ein chemisches Reaktionsmittel, einen UV-Filter und/oder ein thermochromes Material aufweisen. Das chemische Reaktionsmittel kann dazu eingerichtet sein, bei einer chemischen Reaktion Wärme freizusetzen. Die Beschichtung kann auch ein komplementäres chemisches Mittel aufweisen, das mit einem z.B. in einem Reservoir oder der zweiten Hohlkammer vorgehaltenen chemischen Reaktionsmittel unter Wärmefreisetzung reagiert. Das komplementäre chemische Mittel kann beispielsweise ein Calciumoxid sein oder aufweisen.

Es kann vorgesehen sein, alternativ oder zusätzlich auch die Außenseite der Kapsel mindestens teilweise mit einem UV-Filter und/oder einem thermochromen Material zu beschichten. Durch den UV-Filter kann beispielsweise die Haltbarkeit des in der Kapsel aufgenommenen Wirkstoffs bzw. ggf. des Hilfsstoffs erhöht werden. Aufgrund der temperaturabhängigen Farbveränderungen des thermochromen Materials kann dieses als Indikator für die Temperatur im Hohlraum der Kapsel bzw. des Wirkstoffs dienen.

In dem ersten Hohlraum kann eine oder mehrere Mahlkugeln zum Zerkleinern des Wirkstoffs aufgenommen sein. Die Mahlkugel kann ein ferromagnetisches Material aufweisen. Ein Verdampfer, in den die Kapsel einsetzbar ist, kann geeignete Magnete, bspw. Elektromagnete, aufweisen, mittels denen die Mahlkugeln in der Kapsel gezielt bzw. kontrolliert bewegt werden können. Damit kann der in der Kapsel aufgenommene Wirkstoff zerkleinert werden. Durch die Zerkleinerung kann die Oberfläche des Wirkstoffs vergrößert werden, so dass sich bspw. ein besserer Wärmeübergang ergeben kann und/oder die Verdampfung des Wirkstoffs erleichtert wird. Es kann vorgesehen sein, die Mahlkugeln zu beheizen bzw. zu erwärmen, z.B. per Induktionsheizung. Die Mahlkugel kann zur verbesserten Zerkleinerung Schnittkanten aufweisen oder geeignet geformt sein.

Die Innenwand und/oder die Außenwand kann zumindest abschnittsweise aus einem Glas und/oder einem durchsichtigen Kunststoff bestehen. Dadurch kann z.B. der Zustand des Wirkstoffs leicht von außen, d.h. von außerhalb der Kapsel, überprüft werden. Es kann auch vorgesehen sein, dass die Innenwand und/oder die Außenwand Sichtfenster oder dergleichen aufweist. Ist die Außenwand nur abschnittsweise durchsichtig, so kann vorgesehen sein, nur die durchsichtigen Abschnitte mit einem UV-Filter zu beschichten. Weisen die Innenwand und die Außenwand Sichtfenster auf bzw. sind abschnittsweise durchsichtig, so kann vorgesehen sein, die jeweiligen Sichtfenster derart überlappend anzuordnen, dass der erste Hohlraum von außerhalb der Kapsel sichtbar ist.

In dem ersten und/oder zweiten Hohlraum kann eine Heizspule angeordnet sein. Die Heizspule kann eine oder mehrere Spulen aufweisen. Die Heizspule kann alternativ oder zusätzlich eine beheizbare Metallfolie aufweisen. Es kann vorgesehen sein, dass die Heizspule durch einen elektrischen Strom erhitzt wird. Dazu kann eine Außenseite der Kapsel einen elektrischen Kontakt aufweisen, so dass ein elektrischer Strom durch die Heizspule fließen kann. Es kann auch vorgesehen sein, dass die Heizspule durch die Kapselwand hindurch geführt ist. Der in dem ersten Hohlraum aufgenommene Wirkstoff und/oder der in dem zweiten Hohlraum aufgenommene Hilfsstoff kann mit der Heizspule in Kontakt stehen, so dass der Wirkstoff durch die Heizspule erwärmt oder beheizt werden kann.

Die Erfindung betrifft weiter einen Verdampfer zum Verdampfen von Wirkstoffen. Der Verdampfer kann ein Mundstück und eine Verdampfereinheit aufweisen. Der Verdampfer kann eine Kapselaufnahme zur Aufnahme einer erfindungsgemäßen Kapsel aufweisen, wobei die Kapselaufnahme eine erste Kapselöffnungsvorrichtung aufweisen kann und über die erste Kapselöffnungsvorrichtung mit dem Mundstück fluidisch verbunden sein kann, so dass bei aufgenommener Kapsel eine fluidische Verbindung zwischen einem Hohlraum der Kapsel und dem Mundstück durch die erste Kapselöffnungsvorrichtung bereitgestellt werden kann, wobei die Verdampfereinheit zum Verdampfen eines sich bei aufgenommener Kapsel in dem Hohlraum der Kapsel befindlichen Wirkstoffs eingerichtet sein kann. Die Verdampfereinheit kann den Wirkstoff z.B. auf eine Temperatur von 180 - 220 °C erwärmen oder beheizen.

Der Verdampfer ist bevorzugt ein handtragbarer Verdampfer, d.h. bevorzugt derart dimensioniert, dass er bequem in einer menschlichen Hand gehalten werden und/oder mit einer einzigen Hand bedient werden kann. Mit dem Wortlaut "handtragbarer Verdampfer" kann gemeint sein, dass der Verdampfer nicht ortsfest verbunden, sondern mobil ist und/oder Abmessungen und/oder ein Gewicht aufweist, dass er von einer menschlichen Hand gehalten bzw. getragen werden kann.

Die erste Kapselöffnungsvorrichtung kann auf den Deckel einer in den Verdampfer einzusetzenden Kapsel abgestimmt sein, um bei eingesetzter Kapsel eine fluidisch dichte Verbindung zwischen dem ersten und/oder zweiten Hohlraum der Kapsel und dem Mundstück herzustellen. Beispielsweise kann die erste Kapselvorrichtung Mittel zur Perforation des Deckels aufweisen. Die erste Kapselöffnungsvorrichtung kann rohrförmig und/oder durchströmbar sein. Die erste Kapselöffnungsvorrichtung kann einem der Endabschnitt einer Verbindungsleitung zur fluidischen Verbindung einer in die Kapselaufnahme eingesetzten Kapsel mit z.B. dem Mundstück entsprechen. Der Endabschnitt kann der Kapselaufnahme zugewandt sein. Es kann auch vorgesehen sein, dass die Kapselöffnungsvorrichtung der Verbindungsleitung zumindest abschnittsweise entspricht oder mit dieser identisch ist.

Die Verdampfereinheit kann eine Heizplatte in der Kapselaufnahme sein oder aufweisen, so dass bei aufgenommener Kapsel die Heizplatte eine Außenseite, bevorzugt einen Boden der Kapsel, kontaktieren kann.

Die Verdampfereinheit kann ein Reservoir zur Aufnahme eines Fluids sein oder aufweisen, wobei das Reservoir über eine zweite Kapselöffnungsvorrichtung mit der Kapselaufnahme fluidisch verbunden sein kann, so dass bei aufgenommener Kapsel eine fluidische Verbindung zwischen einem Hohlraum der Kapsel und dem Reservoir durch die zweite Kapselöffnungsvorrichtung bereitgestellt werden kann. Das Reservoir kann ein austauschbarer Behälter sein oder aufweisen. Es kann aber auch vorgesehen sein, dass das Reservoir ein in dem Verdampfer aufgenommener und/oder fest montierter Tank ist.

Die zweite Kapselöffnungsvorrichtung kann auf den Deckel einer in den Verdampfer einzusetzenden Kapsel abgestimmt sein, um bei eingesetzter Kapsel eine fluidisch dichte Verbindung zwischen dem Reservoir und dem ersten und/oder zweiten Hohlraum der Kapsel herzustellen. Die zweite Kapselöffnungsvorrichtung kann rohrförmig und/oder durchströmbar sein. Die zweite Kapselöffnungsvorrichtung kann einem Endabschnitt einer Verbindungsleitung zur fluidischen Verbindung z.B. des Reservoirs mit einer in die Kapselaufnahme eingesetzten Kapsel entsprechen. Der Endabschnitt kann der Kapselaufnahme zugewandt sein. Es kann auch vorgesehen sein, dass die Kapselöffnungsvorrichtung der Verbindungsleitung zumindest abschnittsweise entspricht oder mit dieser identisch ist.

Die Verdampfereinheit kann einen oder mehrere Heizdrähte in dem Reservoir und/oder fluidisch zwischen Reservoir und Kapselaufnahme zur Erwärmung des Fluids aufweisen. Das Fluid kann komprimierte Luft oder ein Treibgas sein oder aufweisen. Das Fluid kann den Heizdraht überströmen und dabei erwärmt werden. Das derart erwärmte Fluid kann anschließend in den ersten und/oder zweiten Hohlraum der Kapsel eintreten, den Wirkstoff über- und/oder durchströmen und Wärme z.B. durch Konvektion an den Wirkstoff übertragen. Es kann vorgesehen sein, dass das Fluid Umgebungsluft ist oder aufweist. In diesem Fall kann das Reservoir zur Umgebung der Kapsel geöffnet sein, so dass Umgebungsluft in das Reservoir bzw. den Verdampfer eintreten kann, von dem Heizdraht erhitzt und anschließend in die Kapsel eingeführt werden kann.

Alternativ oder zusätzlich kann das Fluid in dem Reservoir aufgenommen sein, wobei das Fluid ein chemisches Reaktionsmittel sein oder aufweisen kann, so dass durch eine chemische Reaktion des chemischen Reaktionsmittels mit einem komplementären chemischen Mittel Wärme freigesetzt werden kann.

Die Kapselaufnahme, das Reservoir und/oder die zweite Kapselöffnungsvorrichtung können das komplementäre chemische Mittel aufweisen, bevorzugt mit einem solchen beschichtet sein, so dass durch eine chemische Reaktion des chemischen Reaktionsmittels mit dem Fluid Wärme freigesetzt werden kann. Es kann auch vorgesehen sein, dass alternativ oder zusätzlich bei in der Kapselaufnahme aufgenommener Kapsel die Kapsel das komplementäre chemische Mittel aufweist. Beispielsweise kann eine Innenseite der Kapsel und/oder der erste und/oder der zweite Hohlraum mit dem komplementären chemischen Mittel beschichtet sein, so dass das in die Kapsel eingeleitete Fluid bzw. chemische Reaktionsmittel in der Kapsel chemisch reagiert.

Die Verdampfereinheit kann ein Mikrowellengenerator sein oder aufweisen. Weist der Wirkstoff z.B. ein befeuchtetes Cannabisblatt auf, so können Wassermoleküle durch die Mikrowellen angeregt und der Wirkstoff erwärmt werden.

Die Verdampfereinheit kann eine Heizspirale in der Kapselaufnahme sein oder aufweisen. Die Heizspirale kann derart angeordnet sein, dass sie bei in der Kapselaufnahme aufgenommener Kapsel die Außenseite und/oder den Boden der Kapsel kontaktiert. Ist an die Heizspirale ein elektrischer Strom angelegt bzw. fließt ein elektrischer Strom durch die Heizspirale, so kann sich die Heizspirale erwärmen bzw. erhitzen.

Ist in dem ersten und/oder zweiten Hohlraum der in den Verdampfer einzusetzenden Kapsel eine Heizspule angeordnet oder weist die Kapsel eine Heizspule auf, so kann die Kapselaufnahme des Verdampfers geeignete elektrische Kontakte aufweisen, so dass die Heizspule Kapsel elektrisch durchströmt werden kann. Bevorzugt sind die jeweiligen Kontakte an der Außenseite der Kapsel und der Kapselaufnahme derart angeordnet, dass ein Kontakt bei in der Kapselaufnahme eingesetzter Kapsel vorliegt.

Die Verdampfereinheit kann beliebige Kombinationen der beschriebenen Verdampferkonzepte aufweisen. Beispielsweise kann die Verdampfereinheit sowohl eine Heizplatte als auch eine Heizspirale aufweisen.

Der Verdampfer kann ein Betätigungselement aufweisen, wobei die erste und/oder ggf. die zweite Kapselöffnungsvorrichtung Klappen aufweisen kann, die bei Betätigen des Betätigungselements geöffnet werden können. Mit dem Begriff "Klappen" können auch Ventile oder sonstige Vorrichtungen zur Einstellung eines Volumenstroms gemeint sein. Alternativ oder zusätzlich kann die erste und/oder ggf. die zweite Kapselöffnungsvorrichtung bei Betätigen des Betätigungselements in Richtung Kapselaufnahme verfahren werden. Es kann auch vorgesehen sein, dass bei nicht-Alternativ oder zusätzlich kann der Verdampfer Drucksensoren aufweisen, mittels derer beispielsweise ein Ziehen bzw. Saugen am Mundstück detektiert werden kann, wobei die Klappen bei erkanntem Unterdruck und/oder Ziehen am Mundstück geöffnet werden können und/oder die Kapselöffnungsvorrichtungen verfahren werden können. Es kann vorgesehen sein, dass bei nicht-betätigtem Betätigungselement bzw. einem Stopp des Betätigens und/oder einem nicht mehr durch das Ziehen am Mundstück anliegendem Unterdruck die erste und/oder zweite Kapselöffnungsvorrichtung von der Kapsel weg- bzw. zurückgefahren wird, so dass die Kapselöffnungsvorrichtung nicht mehr in das Kapselinnere hineinragt. Es kann vorgesehen sein, dass der Deckel beim weg- bzw. zurückfahren der Kapselöffnungsvorrichtung geschlossen wird, insbesondere fluidisch dicht wird.

Alternativ oder zusätzlich kann vorgesehen sein, dass bei Betätigen des Betätigungselements und/oder bei erkanntem Unterdruck die Verdampfereinheit angesteuert wird, so dass der Wirkstoff und/oder der Hilfsstoff verdampft wird. Das Betätigungselement kann an einer Außenseite des Verdampfers, z.B. an einem Griff, angeordnet sein. Das Betätigungselement kann ein Taster, ein Schieber, ein Druckknopf, ein Drehknopf, ein haptisches Element oder dergleichen sein oder aufweisen.

Es kann vorgesehen sein, dass bereits während oder nach dem Einsetzen der Kapsel in die Kapselaufnahme eine fluidische Verbindung zwischen dem Hohlraum der Kapsel und dem Mundstück durch die erste Kapselöffnungsvorrichtung und/oder ggf. zwischen dem Reservoir und dem Hohlraum hergestellt wird. Beispielsweise kann der Deckel der Kapsel bei einer Bewegung der Kapsel beim Einsetzen der Kapsel perforiert werden. Wird die Kapsel z.B. in ihrem eingesetzten Zustand durch einen verschwenkbaren Kapselhalter, z.B. eine verschwenkbare Platte, in dem Verdampfer bzw. der Kapselaufnahme gehalten, so kann beim Schließen des Kapselhalters die Kapsel derart geführt bewegt werden, dass der Deckel z.B. perforiert wird oder auf dem Deckel vorgesehene Ventile geeignet mit rohrartigen Verbindungsleitungen oder dergleichen dichtend abschließen oder an diese angeschlossen werden.

Der Verdampfer kann eine fluidisch zwischen Kapselaufnahme und Mundstück angeordnete Kühleinheit aufweisen, so dass von dem Verdampfer verdampfter Wirkstoff gekühlt werden kann, bevorzugt auf eine Temperatur von 10 - 30°C, besonders bevorzugt auf eine Temperatur von 15 - 25°C. Die Kühleinheit kann beispielsweise eine Kühlschlange und/oder Kühlrippen aufweisen. Es kann auch eine Konvektionskühlung z.B. durch Luftströme oder dergleichen vorgesehen sein. Die Kühleinheit kann einen Lüfter, Ventilator oder dergleichen aufweisen.

Der Verdampfer kann einen dem Mundstück fluidisch vorgelagerten Speicher aufweisen, in welchem von dem Verdampfer verdampfter Wirkstoff gesammelt werden kann. Es kann vorgesehen sein, dass der Speicher beheizbar ist und/oder gekjühlt werden kann und/oder thermisch isoliert ist. Damit kann eine gewisse Menge bereits verdampften Wirkstoffs vorgehalten werden, so dass z.B. Unterbrechungen des Verdampfens des Wirkstoffs überbrückt werden können. Je nach Art der verwendeten Verdampfereinheit kann eine zeitliche Differenz zwischen Beginn der Erwärmung des Wirkstoffs und Bereitstellen des verdampften Wirkstoffs vorliegen, die ebenfalls durch in dem Speicher vorgehaltenen, bereits verdampften Wirkstoff überbrückt werden kann.

Das Mundstück kann einen Zerstäuber zum Zerstäuben von verdampftem Wirkstoff und/oder Hilfsstoff in Tröpfchen aufweisen. Der Durchmesser der jeweiligen Tröpfchen und/oder der mittlere Durchmesser der Tröpfchenverteilung kann zwischen 0,5 bis 500 µm, bevorzugt zwischen 0,5 bis 50 µm und besonders bevorzugt zwischen 0,5 bis 5 µm betragen. Damit kann der verdampfte und zerstäubte Wirkstoff und/oder Hilfsstoff bzw. das entsprechende Aerosol beim Einatmen bis in die feinsten Verästelungen der Lunge oder der Atmungswege gelangen. Der Zerstäuber kann ein Düsenvernebler, ein Ultraschallvernebler und/oder ein Membranvernebler sein oder aufweisen.

Das Mundstück, der Zerstäuber und/oder die erste und/oder ggf. die zweite Kapselöffnungsvorrichtung kann eine Venturi-Düse sein oder aufweisen.

Der Verdampfer kann mindestens einen dem Mundstück fluidisch vorgelagerten Filter aufweisen, wobei der Filter bevorzugt an dem Mundstück angeordnet sein kann.

Der Verdampfer kann eine Steuer- und/oder Regelungseinheit und Sensoren zur Steuerung und Regelung der Temperatur der Verdampfereinheit aufweisen. Die Steuer-und/oder Regelungseinheit kann auch dazu vorgesehen sein, die Temperatur der Kühleinheit zu steuern und/oder zu regeln. Die Sensoren können in der Kapselaufnahme, den Hohlräumen der Kapsel, dem Reservoir, der Kühleinheit, der Verdampfereinheit, dem Speicher, dem Mundstück und/oder in den jeweiligen Verbindungsleitungen angeordnet sein. Die Sensoren können dazu eingerichtet sein, die Temperatur, die Strömungsgeschwindigkeit bzw. den Volumenstrom, den Druck, die Gaszusammensetzung oder die Zusammensetzung des verdampften Wirkstoffs zu erfassen und an die Steuer- und/oder Regelungseinheit weiterzuleiten. Die Steuer-und/oder Regelungseinheit kann einen Prozessor, einen Mikrocontroller, einen integrierten Schaltkreis, ein FPGA oder dergleichen aufweisen. Der Verdampfer kann ein Betätigungselement oder einen Regler zum Einstellen des Volumenstroms des verdampften Wirkstoffs aufweisen. Das Betätigungselement oder der Regler kann z.B. ein Drehknopf, ein Taster, ein Schieber oder dergleichen sein. Das Betätigungselement oder der Regler kann an einer Außenseite des Verdampfers angeordnet sein.

Der Zerstäuber kann einen labyrinthartigen Kanal mit mindestens einer Abrisskante aufweisen, so dass durch den Kanal strömende Tröpfchen größer als eine maximale Tröpfchengröße abgeschieden werden können.

Der Verdampfer kann einen Akkumulator, eine Batterie oder dergleichen aufweisen, um die Verdampfereinheit, die Sensoren und/oder die Steuer- und/oder Regeleinheit oder andere im Verdampfer aufgenommene, Strom benötigende Komponenten mit Strom bzw. Energie zu versorgen.

Der Verdampfer kann einen Fingerabdrucksensor und/oder eine Vorrichtung zur Benutzeridentifizierung aufweisen. Der Fingerabdrucksensor kann an dem Gehäuse derart angeordnet sein, dass bei einer üblichen Handhabung bei der Benutzung, oder bevorzugt einhändigen Benutzung, der Fingerabdrucksensor einen Fingerabdruck erfasst und/oder erfassen kann. Beispielsweise kann der Fingerabdrucksensor derart angeordnet sein, dass bei einhändiger Benutzung des Verdampfers und Betätigung des Betätigungselements ein Finger derselben Hand auf dem Fingerabdrucksensor aufliegt. Damit kann sichergestellt werden, dass nur berechtigte Benutzer den Verdampfer benutzen können. Die Informationen bezüglich Benutzeridentifikation können z.B. in der Steuer-und/oder Regelungseinheit gespeichert sein.

Es kann vorgesehen sein, dass die Außenseite des Deckels und oder des Bodens der Kapsel einen Code, beispielsweise einen QR-Code-Code, einen alphanumerischen Code, einen Barcode und/oder ein Piktogramm, und/oder einen RFID-Chip aufweist. Das Piktogramm kann auch eine Anordnung, z.B. Aneinanderreihung, einzelner Piktogramme sein. Der Code kann aber auch alternativ oder zusätzlich an einer Seitenwand der Kapsel angeordnet sein. Der Verdampfer kann eine Leseeinheit aufweisen, wobei die Leseeinheit dazu eingerichtet sein kann, bei eingesetzter Kapsel einen auf einer Außenseite der Kapsel angebrachten Code, bevorzugt einen QR-Code, einen alphanumerischen Code und/oder ein Piktogramm, und/oder einen RFID-Chip zu erfassen und/oder auszulesen. Die Leseeinheit kann bevorzugt in der Kapselaufnahme angeordnet sein. Die Leseeinheit kann Die Leseeinheit kann mit der Steuer-und/oder Regelungseinheit im Datenaustausch stehen. Der Code kann z.B. Informationen bezüglich des in der Kapsel aufgenommen Wirkstoffs bzw. Wirkstoffe und/oder der Verdampfungstemperatur enthalten. Es kann vorgesehen sein, die Informationen bezüglich Verdampfungstemperatur zur Steuerung des Heizelements zu verwenden. Weist der Verdampfer einen Fingerabdrucksensor oder eine sonstige Vorrichtung zur Benutzeridentifizierung auf, so kann vorgesehen sein, nur dann den Wirkstoff bzw. die Wirkstoffe in der Kapsel zu verdampfen, wenn der durch den Fingerabdrucksensor oder sonstwie identifizierte Benutzer dazu berechtigt ist. Die Überprüfung der Berechtigung kann z.B. durch die Steuer-und/oder Regelungseinheit durch Abgleich mit den Benutzerinformationen vorgenommen werden. Die Leseeinheit kann dazu eingerichtet sein, den Code optisch, z.B. mit einer Kamera, zu erfassen. Die Leseeinheit kann dazu eingerichtet sein, den Code optisch, z.B. mit einer Kamera und optional einer Beleuchtung, zu erfassen. Die Leseeinheit kann dazu eingerichtet sein, den RFID-Chip elektromagnetisch auszulesen. Mit dem Begriff "RFID-Chip" kann auch ein RFID-Transponder gemeint sein.

Die Erfindung betrifft weiter ein Verdampfersystem zum Verdampfen eines Wirkstoffs, aufweisend einen erfindungsgemäßen Verdampfer und eine in der Kapselaufnahme des Verdampfers aufgenommene erfindungsgemäße Kapsel. Das Verdampfersystem kann zusätzlich eine Lade-oder Dockingstation zur Aufnahme des Verdampfers umfassen.

Die Erfindung wird anhand der nachfolgenden Figuren weiter erläutert. Dabei zeigt:
- Figur 1: eine Ausführungsform einer erfindungsgemäßen Kapsel;
- Figur 2: eine weitere Ausführungsform einer erfindungsgemäßen Kapsel;
- Figur 3: noch eine weitere Ausführungsform einer erfindungsgemäßen Kapsel;
- Figur 4: eine weitere Ausführungsform einer erfindungsgemäßen Kapsel;
- Figur 5: ein Verdampfersystem mit einem erfindungsgemäßen Verdampfer und einer in der Kapselaufnahme aufgenommenen erfindungsgemäßen Kapsel;
- Figur 6: ein weiteres Verdampfersystem mit einem erfindungsgemäßen Verdampfer und einer in der Kapselaufnahme aufgenommenen erfindungsgemäßen Kapsel;
- Figur 7: noch ein weiteres Verdampfersystem mit einem erfindungsgemäßen Verdampfer und einer in der Kapselaufnahme aufgenommenen erfindungsgemäßen Kapsel;
- Figur 8: noch ein weiteres Verdampfersystem mit einem erfindungsgemäßen Verdampfer und einer in der Kapselaufnahme aufgenommenen erfindungsgemäßen Kapsel;
- Figur 9: noch ein weiteres Verdampfersystem mit einem erfindungsgemäßen Verdampfer und einer in der Kapselaufnahme aufgenommenen erfindungsgemäßen Kapsel;
- Figur 10: noch ein weiteres Verdampfersystem in perspektivischer Ansicht mit einem erfindungsgemäßen Verdampfer und einer in der Kapselaufnahme aufgenommenen erfindungsgemäßen Kapsel; und
- Figur 11: eine schematische Darstellung der Steuer- und/oder Regelungseinheit sowie der damit verbundenen Komponenten.

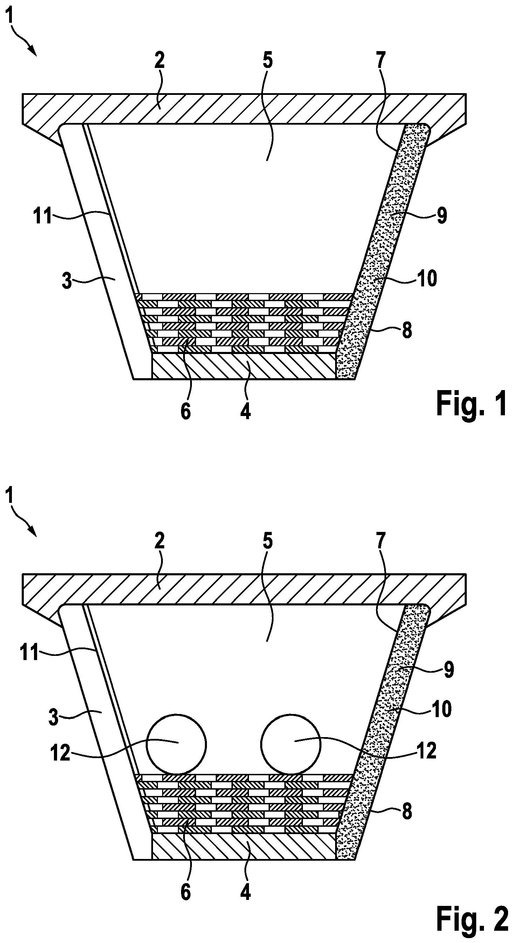

Figur 1 zeigt eine Ausführungsform einer erfindungsgemäßen Kapsel 1. Die Kapsel 1 weist einen Deckel 2, Seitenwände 3 und einen Boden 4 auf. Die Innenwände 7 der Seitenwände 3 und die Innenseite des Deckels 2 und des Bodens 4 bilden einen ersten Hohlraum 5 im Inneren der Kapsel 1. Die Seitenwände 3 sind doppelwandig ausgeführt, mit einer dem ersten Hohlraum 5 zugewandten Innenwand 7 und einer die Außenseite der Seitenwand 3 bildenden Außenwand 8. Zwischen Innenwand 7 und Außenwand 8 ist zumindest abschnittsweise ein zweiter Hohlraum 9 ausgebildet. Der Boden 4 kann ebenfalls doppelwandig ausgeführt sein, so dass auch im Boden zumindest abschnittsweise ein zweiter Hohlraum 9 ausgebildet sein kann. In dem ersten Hohlraum 5 ist ein Wirkstoff 6 aufgenommen. In dem zweiten Hohlraum ist ein Hilfsstoff 10 aufgenommen. Die Innenwand 7 kann zumindest teilweise mit einer Beschichtung 11 beschichtet sein. Alternativ oder zusätzlich kann auch die Innenseite des Deckels 2 und/oder des Bodens 4 beschichtet sein. Der Boden 4 kann zumindest abschnittsweise aus einem Material bestehen oder ein solches aufweisen, das verglichen mit dem Material der Seitenwand eine hohe Wärmeleitfähigkeit aufweist. Der Wirkstoff 6 kann ein pflanzlicher Wirkstoff sein. Bevorzugt ist der Wirkstoff 6 ein Cannabinoid oder weist ein solches auf. Beispielsweise kann der Wirkstoff 6 eine Cannabisblüte, ein Cannabisextrakt oder ein Cannabisöl sein oder aufweisen. Der Hilfsstoff 10 kann einen mit dem Wirkstoff 6 identischen Wirkstoff aufweisen. Beispielsweise können sowohl der Wirkstoff 6 als auch der Hilfsstoff 10 gemahlene Cannabisblüten sein oder aufweisen. Der Hilfsstoff 10 kann aber auch einen von dem Wirkstoff 6 verschiedenen Wirkstoff aufweisen, der mit dem Wirkstoff 6 z.B. zu einer neuen Wirkung zusammenwirken kann oder dessen Wirkung verstärken oder modifizieren kann. Der Hilfsstoff 10 kann ein chemisches Reaktionsmittel aufweisen. Das chemische Reaktionsmittel kann dazu eingerichtet sein, mit einem komplementären chemischen Mittel exotherm zu reagieren. Der Hilfsstoff 10 kann einen Aromastoff aufweisen, um beispielsweise dem verdampften Wirkstoff 6 einen Fruchtgeschmack zu verleihen. Der Hilfsstoff 10 kann aber auch alternativ oder zusätzlich einen Lebensmittelzusatzstoff oder Nahrungsergänzungsmittel sein oder aufweisen.

Die Innenwand 7 kann zumindest abschnittsweise semi-permeabel, perforierbar und/oder porös sein oder Sollbruchstellen aufweisen. Dadurch kann eine fluidische Verbindung zwischen erstem und zweitem Hohlraum 5, 9 hergestellt sein oder werden. Weist die Seitenwand 3 eine oder mehrere Zwischenwände auf, so können auch die Zwischenwände zumindest abschnittsweise semi-permeabel, perforierbar und/oder porös sein oder Sollbruchstellen aufweisen.

Die Kapsel 1 kann an einer Innenseite, z.B. der Innenwand 7 oder des Deckels 2 oder des Bodens 4, zumindest abschnittsweise eine Beschichtung 11 aufweisen. Die Beschichtung 11 kann z.B. ein komplementäres chemisches Mittel aufweisen, das mit dem chemischen Reaktionsmittel exotherm reagieren kann. Es kann auch vorgesehen sein, dass die Innenseiten der ersten und/oder zweiten Hohlräume 5, 9 zumindest abschnittsweise eine Beschichtung 11 aufweisen. In mindestens einer Ausführungsform weist mindestens eine Innenseite des ersten Hohlraums 5 zumindest abschnittsweise eine Beschichtung mit einem komplementäres chemisches Mittel auf, wobei der Hilfsstoff 10 in dem zweiten Hohlraum 9 ein chemisches Reaktionsmittel aufweist und in dem zweiten Hohlraum 9 ein Treibgas aufgenommen ist. Wird der zweite Hohlraum 9 zum ersten Hohlraum 5 geöffnet, z.B. durch Perforierung der Innenwand 7, so kann das chemische Reaktionsmittel durch das Treibgas in den ersten Hohlraum 5 eingebracht werden und dort mit dem komplementären chemischen Mittel exotherm reagieren, so dass der Wirkstoff 6 erwärmt und verdampft wird.

Bevorzugt kann die Kapsel 1 luftundurchlässig sein, d. h. bei geschlossenem Deckel 2 kann der erste und/oder der zweite Hohlraum 5, 9 von einer Umgebung der Kapsel fluidisch getrennt sein, so dass z.B. keine Luft aus der Umgebung in die jeweiligen Hohlräume eintreten kann. Der Deckel 2 kann perforierbar sein. Alternativ kann der Deckel 2 porös und/oder semi-permeabel sein. Es kann aber auch sein, dass der Deckel 2 Sollbruchstellen und/oder Ventile aufweist. Damit kann eine fluidische Verbindung des ersten und/oder zweiten Hohlraums 5, 9 durch den Deckel 2 zu der Umgebung der Kapsel 1 hergestellt werden.

Die Kapsel 1, insbesondere die Seitenwände 3 bzw. die Innenwand 7 und/oder die Außenwand 8, kann zumindest teilweise aus Glas und/oder einem durchsichtigen Kunststoff bestehen oder aufweisen. Damit kann z.B. der Zustand des Wirkstoffs 6 und/oder des Hilfsstoff 10 von außen ohne Öffnen der Kapsel 1 sichtbar sein bzw. untersucht werden. Es kann vorgesehen sein, dass die Innenwand 7, die Außenwand 8, der Deckel 2 und/oder der Boden 4 Sichtfenster aufweisen. Weisen sowohl die Innenwand 7 als auch die Außenwand 8 Sichtfenster auf, so können diese von einer Kapselachse aus betrachtet in radialer Richtung überlappend angeordnet sein, so dass der erste Hohlraum 5 durch den zweiten Hohlraum 9 hindurch von außen sichtbar sein kann. Es kann vorgesehen sein, dass die Außenseite der Kapsel, insbesondere ggf. vorhandene Sichtfenster, zumindest teilweise mit einem UV-Filter beschichtet ist. Die Außenseite der Kapsel 1 kann auch zumindest teilweise mit einem thermochromen Material beschichtet sein.

Die Kapsel kann im Wesentlichen zylinderförmig, konisch, prismenförmig, zylinderstumpfförmig pyramidal, pyramidenstumpfförmig, kubisch, quaderförmig, paraboloid oder dergleichen sein.

Figur 2 zeigt eine weitere Ausführungsform einer erfindungsgemäßen Kapsel 1. Verglichen mit der in Figur 1 dargestellten Ausführungsform weist die Kapseln eine oder mehrere Mahlkugeln 12 auf. Die Mahlkugeln 12 können aus einem ferromagnetischen Material bestehen, so dass sie beispielsweise durch Elektromagnete gezielt bewegt werden können. Dadurch kann der in dem ersten Hohlraum 5 der Kapsel 1 aufgenommene Wirkstoff 6 durch die Bewegungen der Mahlkugeln 12 zerkleinert werden. Es kann auch vorgesehen sein, dass die Mahlkugeln 12 beispielsweise durch Induktion erwärmt werden können, um den Wirkstoff 6 zu verdampfen oder das Verdampfen des Wirkstoffs 6 zu unterstützen. Die Mahlkugeln 12 können Schnittkanten oder dergleichen aufweisen.

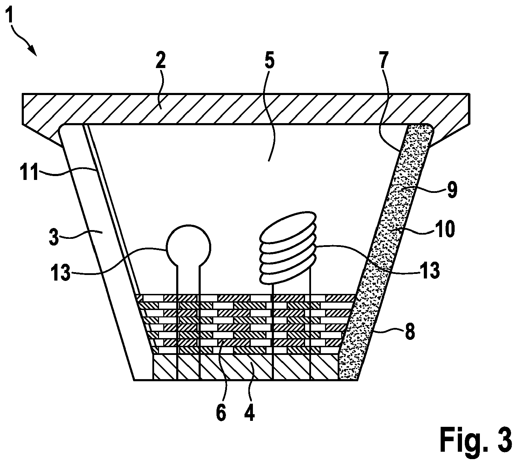

Figur 3 zeigt eine weitere Ausführungsform einer erfindungsgemäßen Kapsel 1. Verglichen mit der in Figur 1 dargestellten Ausführungsform weist die Kapsel eine oder mehrere Heizspulen 13 auf. Die Heizspule 13 kann die in der Figur dargestellt beispielsweise spiralförmig oder gewunden sein. Die Heizspule 13 kann aber auch als Hitzdraht oder als Metallfolie ausgeführt sein. Fließt ein elektrischer Strom durch die Heizspule 13, so kann sich diese erwärmen und damit den Wirkstoff 6 und/oder den Hilfsstoff 10 verdampfen. Die Kapsel 1 kann an einer Außenseite, beispielsweise am Boden 4, Kontakte aufweisen, um einen elektrischen Strom durch die Heizspule 13 zu führen. Es kann vorgesehen sein, dass der Verdampfer 100 in der Kapselaufnahme 103 dazu korrespondierende Kontakte aufweist, so dass bei eingesetzter Kapsel 1 ein elektrischer Kontakt hergestellt werden und ein Strom durch die Heizspule 13 fließen kann.

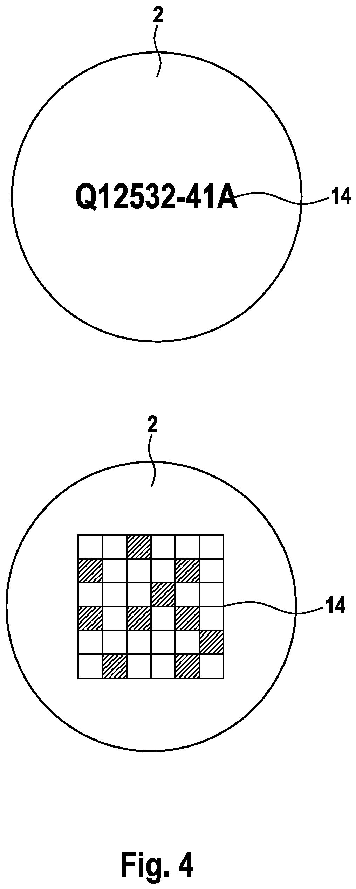

Figur 4 zeigt den Deckel 2 einer weiteren Ausführungsform einer erfindungsgemäßen Kapsel 1 in einer Draufsicht. Der Deckel 2 kann im Wesentlichen kreisförmig, rechteckig, elliptisch oder polygonal sein. Auf dem Deckel 2 ist ein Code 14, beispielsweise ein alphanumerischen Code, ein QR-Code, ein Barcode und/oder ein Piktogramm, angeordnet. Der Code 14 kann z.B. Informationen bezüglich des Inhalts der Kapsel 1, beispielsweise bezüglich der Zusammensetzung und/oder Menge des Wirkstoffs 6 und/oder des Hilfsstoff 10 aufweisen bzw. enthalten Der Code 14 kann alternativ oder zusätzlich Informationen bezüglich des Verdampfungsprozesses, beispielsweise einer Verdampfungstemperatur und/oder eines zeitlichen Temperaturverlaufs enthalten. Alternativ oder zusätzlich kann der Code 14 Informationen bezüglich eines Verdampfertyps, eines individuellen Verdampfers und/oder eines Benutzers enthalten, so dass eine bestimmte Kapsel nur mit einem übereinstimmenden Verdampfertyp und/oder eines individuellen Verdampfers und/oder nur von einem bestimmten Benutzer verwendet werden kann. Alternativ oder zusätzlich können die jeweiligen Informationen auch auf einem an der Außenseite des Deckels 2 der Kapsel 1 angeordneten RFID-Chip, RFID-Transponder oder dergleichen gespeichert sein. Der Verdampfer 100 kann eine entsprechende Leseeinheit 120 aufweisen. Die Leseeinheit 120 kann beispielsweise derart in der Kapselaufnahme 103 angeordnet sein, dass bei eingesetzter Kapsel 1 der Code 14 und/oder der RFID-Chip erfasst bzw. ausgelesen werden kann. Die Leseeinheit 120 kann die jeweiligen Informationen beispielsweise optisch oder elektromagnetisch erfassen. Die Leseeinheit 120 kann mit der Steuer-und/oder Regelungseinheit 116 des Verdampfers 100 zum Datenaustausch verbunden sein. Damit kann die Steuer- und/oder Regelungseinheit 116 basierend auf den von der Leseeinheit 120 erfassten Informationen beispielsweise die Verdampfungstemperatur und/oder deren zeitlichen Verlauf einstellen. Werden beispielsweise Abweichungen des Verdampfertyps, des individuellen Verdampfers und/oder des Benutzers von den im Code 14 oder dem RFID-Chip enthaltenen Informationen durch die Steuer- und/oder Regelungseinheit 116 festgestellt, so kann vorgesehen sein, dass die Steuer- und/oder Regelungseinheit 116 die Verdampfereinheit 102 nicht ansteuert bzw. betätigt, so dass der Wirkstoff 6 und/oder der Hilfsstoff 10 nicht oder nur teilweise verdampft wird, und/oder keine fluidische Verbindung zwischen erstem und/oder zweitem Hohlraum 5, 9 und Mundstück 101 herstellt. Es kann beispielsweise auch vorgesehen sein, dass die Steuer- und/oder Regelungseinheit 116 im Code oder RFID-Transponder enthaltene Informationen bzgl. des Wirkstoffs 6 und/oder Hilfsstoffs 10 mit einer mit der Steuer- und/oder Regeleinheit 116 verbundenen oder zugeordneten Datenbank zur Berechtigungskontrolle abgleicht. Insbesondere bei der medizinischen Anwendung kann damit eine Fehl- oder Überdosierung verhindert werden. Insbesondere kann damit auch sichergestellt werden, dass ein Patient nur die für ihn vorgesehene Dosierung, Mischung und/oder Wirkstoffe verdampfen bzw. einatmen kann.

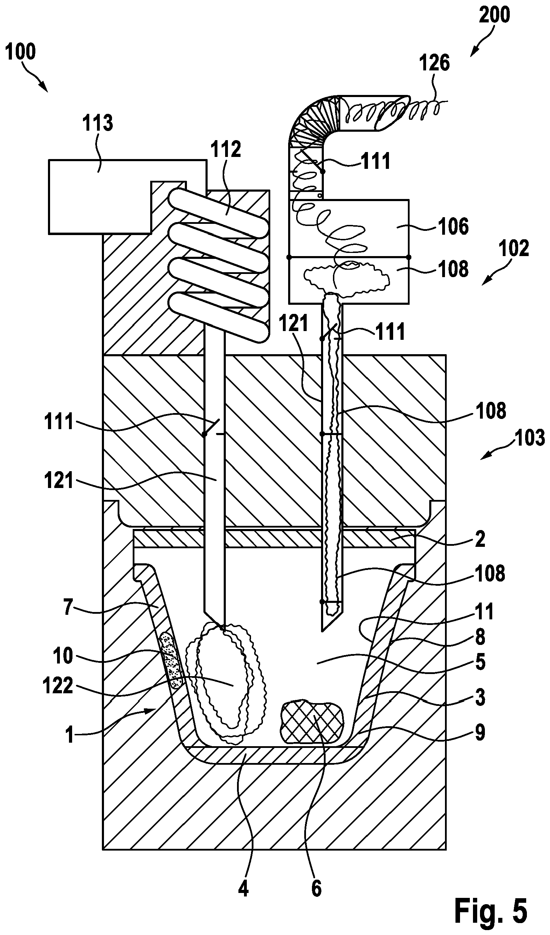

Figur 5 zeigt einen Teilausschnitt eines erfindungsgemäßen Verdampfers 100 mit einer in der Kapselaufnahme 103 eingesetzten erfindungsgemäßen Kapsel 1. Bei dem in Figur 5 gezeigten Ausführungsbeispiel ist der Deckel 2 der Kapsel 1 durch die erste Kapselöffnungsvorrichtung 104 und die zweite Kapselöffnungsvorrichtung 107 perforiert bzw. durchstoßen. Die Kapselöffnungsvorrichtungen 104, 107 der Figur 5 entsprechen dabei Endstücken von Verbindungsleitungen 121. Durch die erste Kapselöffnungsvorrichtung 104 ist der erste Hohlraum 5 der Kapsel 1 über die Kühlvorrichtung 112 mit dem Speicher 113 und damit mittelbar mit dem nicht in Figur 5 gezeigten Mundstück 101 fluidisch verbunden. Bei dem in Figur 5 gezeigten Ausführungsbeispiel ist der erste Hohlraum 5 durch die zweite Kapselöffnungsvorrichtung 107 mit dem Reservoir 106 fluidisch verbunden. Die Kapselöffnungsvorrichtungen 104, 107 können beispielsweise eine Schneidkante aufweisen oder abgeschrägt sein, um die Perforierung des Deckels 2 zu erleichtern. Es kann vorgesehen sein, dass die erste und/oder zweite Kapselöffnungsvorrichtung 104, 107 ortsfest in dem Verdampfer angeordnet ist. Beim Einsetzen der Kapsel 1 in die Kapselaufnahme 103 kann dabei durch die Bewegung der Kapsel 1 relativ zu der ersten und/oder zweiten Kapselöffnungsvorrichtung 104, 107 der Deckel 2 durch die erste und/oder zweite Kapselöffnungsvorrichtungen 104, 107 perforiert werden. Es kann aber auch vorgesehen sein, dass die erste und/oder zweite Kapselöffnungsvorrichtung 104, 107 in dem Verdampfer 100 beweglich angeordnet sind, so dass diese gegen die in die Kapselaufnahme 103 eingesetzte Kapsel 1 beweglich sind, so dass durch die Bewegung der ersten und/oder zweiten Kapselöffnungsvorrichtung 104, 107 in Richtung der in der Kapselaufnahme 103 eingesetzten Kapsel 1 der Deckel 2 perforiert wird. Es kann vorgesehen sein, dass die Bewegung der ersten und/oder zweiten Kapselaufnahme 104, 107 in Richtung der Kapselaufnahme 103 durch eine Betätigung des Betätigungselements 110 initiiert wird und/oder bei betätigtem Betätigungselement 110 erfolgt.

Es kann vorgesehen sein, dass die erste und/oder zweite Kapselöffnungsvorrichtung 104, 107 derart geformt oder eingerichtet ist, dass beim Einsetzen der Kapsel 1 in die Kapselaufnahme 103 die Innenwand 7 durch die erste und/oder zweite Kapselöffnungsvorrichtung 104, 107 derart geöffnet wird, dass sich eine fluidische Verbindung zwischen dem ersten Hohlraum 5 und dem zweiten Hohlraum 10 ergibt. Beispielsweise kann die Innenwand 7 dafür geeignete Sollbruchstellen aufweisen. Alternativ oder zusätzlich kann die Innenwand 7 zumindest teilweise semi-permeabel sein, so dass z.B. der verdampften Hilfsstoff 10 die Innenwand in Richtung erster Hohlraum 5 passieren kann bzw. durch die semi-permeable Innenwand 7 in den Hohlraum eintreten kann.

Bei dem in Figur 5 gezeigten Ausführungsbeispiel weist die Verdampfereinheit 2 das Reservoir 106 auf. In dem Reservoir 106 kann ein chemisches Reaktionsmittel vorgehalten bzw. gespeichert sein. Die Kapselinnenwand 7, die Innenseite des Deckels 2 und oder des Bodens 4 und/oder die Verbindungsleitung zwischen ersten Hohlraum 5 und Reservoir 106 kann derart beschichtet sein, dass das chemische Reaktionsmittel mit der Beschichtung exotherm reagiert, so dass Wärme frei wird. Durch die freigegebene Wärme kann der im ersten Hohlraum 5 aufgenommene Wirkstoff 6 und/oder oder der im zweiten Hohlraum 9 aufgenommene Hilfsstoff 10 verdampft werden. Der verdampfte Wirkstoff 6 und/oder der verdampfte Hilfsstoff 10 kann durch die erste Kapselöffnungsvorrichtung 104 in den Speicher 113 überführt und ggf. durch die Kühlvorrichtung 112 gekühlt werden. Die Verbindungsleitungen 121 können Klappen 111 und/oder Ventile aufweisen, um die fluidische Verbindung zwischen dem Inneren der Kapsel und dem Speicher 113 bzw. dem Reservoir 106 zu verschließen oder freizugeben. Die Klappen 111 und/oder die Ventile können von einer Steuer-und/oder Regelungseinheit 116 gesteuert werden. So kann beispielsweise vorgesehen sein, dass nach einer Betätigung eines Betätigungselements 110 die Steuer-und/oder Regelungseinheit 116 die Klappen 111 öffnet. Selbstverständlich können die jeweiligen Klappen 111 individuell von der Steuer-und/oder Regelungseinheit 116 angesteuert werden, z.B. zeitlich versetzt geöffnet und/oder geschlossen werden.

Das Reservoir 106 kann beispielsweise eine austauschbare Kartusche sein. Das Reservoir 106 kann aber auch ein fest in dem Verdampfer 100 verbauter, bevorzugt von außen befüllbarer, Tank oder Behälter sein. Die Einbringung des in dem Reservoir 106 gespeicherten Fluids durch die zweite Kapselöffnungsvorrichtung 107 in die Kapsel kann beispielsweise, ggf. bei geöffneter Klappe 111, durch die Schwerkraft erfolgen. Es kann aber auch vorgesehen sein, die Einbringung durch einen Überdruck zu erzeugen oder zu unterstützen, beispielsweise durch eine nicht in Figur 5 gezeigte Pumpe, Verdichter oder dergleichen. Es kann auch vorgesehen sein, den Überdruck z.B. durch einen Druckluftbeaufschlagung, ein Treibgas oder dergleichen zu erzeugen. Beispielsweise kann Druckluft oder ein Treibgas über eine weitere Verbindungsleitung 126 wie in Figur 5 angedeutet in das Reservoir 106 eingeleitet werden wobei auch die weitere Verbindungsleitung 126 eine von der Steuer-und/oder Regelungseinheit 116 ansteuerbare Klappe 111 aufweisen kann. Es kann aber auch vorgesehen sein, dass in dem Reservoir kein chemisches Reaktionsmittel, sondern beispielsweise Druckluft oder ein Treibgas gespeichert ist. In diesem Falle ist das Reservoir 106 bevorzugt eine austauschbare Druckluftkartusche. Durch einen Heißdraht 108 kann die Druckluft oder das Treibgas erwärmt und nach Einbringung in die Kapsel 1 zum Verdampfen des Wirkstoffs 6 und/oder des Hilfsstoff 10 eingerichtet sein. Der Heißdraht 108 kann dabei in dem Reservoir 106, der Verbindungsleitung 121 und/oder der zweiten Kapselöffnungsvorrichtung 107 angeordnet sein. Bevorzugt wird die Druckluft derart erwärmt, dass ihre Temperatur unter der Verbrennungstemperatur des Wirkstoffs 6 und/oder des Hilfsstoffs 10 liegt. Damit kann der Wirkstoff 6 und/oder des Hilfsstoff 10 verdampfen, trotz Luftzufuhr aber nicht verbrennen. Neben Luft können aber auch andere geeignete Fluide und/oder Gase verwendet werden. Wird Luft verwendet, so kann vorgesehen sein, diese nicht in einem Reservoir 106 vorzuhalten bzw. bereitzustellen, sondern der Umgebung zu entnehmen und/oder aus der Umgebung einzusaugen bzw. in dem Verdampfer 100 einzuführen. Dazu kann der Verdampfer 100 einen Ventilator oder dergleichen aufweisen.. Die Umgebungsluft kann anschließend durch den Heißdraht 108 erwärmt und über die zweite Kapselöffnungsvorrichtung 107 in die Kapsel 1 eingeführt werden.

Die Kühlvorrichtung 112 kann eine Kühlschlange sein oder aufweisen. Alternativ oder zusätzlich kann die Kühlvorrichtung 112 Kühlrippen aufweisen. Es kann auch vorgesehen sein, dass die Kühlvorrichtung 112 ein Gebläse, beispielsweise ein Ventilator aufweist. Alternativ oder zusätzlich kann die Kühlvorrichtung 112 eine Verdunstungskühlung aufweisen. Es kann vorgesehen sein, dass die Kühlvorrichtung 112 von der Steuer-und/oder Regelungseinheit 116 ansteuerbar ist so dass die Kühlleistung variabel bzw. einstellbar ist und geregelt bzw. gesteuert werden kann.

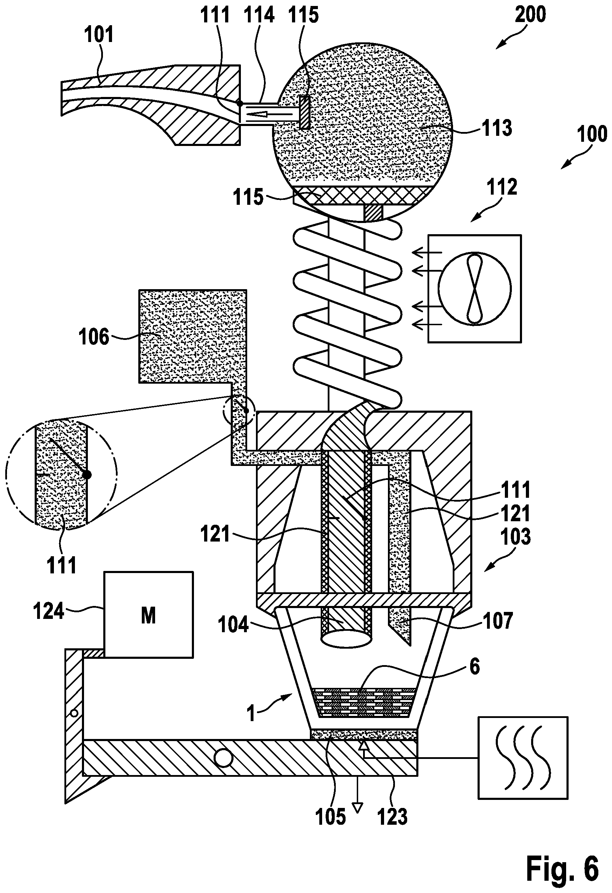

Figur 6 zeigt eine weitere Ausführungsform eines erfindungsgemäßen Verdampfers 100 mit einer in der Kapselaufnahme 103 aufgenommenen erfindungsgemäßen Kapsel 1. Die Verdampfereinheit 102 weist ein mit Bezug auf Figur 5 oben beschriebenes Reservoir 106 mit einem chemischen Reaktionsmittel auf. Zusätzlich weist die Verdampfereinheit 102 eine Heizplatte 105 auf, die den Boden 4 der Kapsel 1 kontaktiert. Der Boden 4 der Kapsel 1 besteht bevorzugt aus einem Material mit einer hohen Wärmeleitfähigkeit, so dass Wärme von der Heizplatte 105 an die dem Kapselinneren zugewandten Innenseite des Bodens 4 geleitet werden kann, so dass der Wirkstoff 6 und der nicht in Figur 6 gezeigte Hilfsstoff 10 verdampft werden können. Die Heizplatte 105 kann von der Steuerung-und/oder Regelungseinheit 116 angesteuert werden, so dass die Temperatur der Heizplatte 105 steuer- bzw. regelbar ist. Die Heizplatte 105 kann in der Kapselaufnahme 103 derart angeordnet sein, dass sie bei eingesetzter Kapsel 1 die Außenwand 8, den Deckel 2 oder den Boden 4 der Kapsel 1 kontaktiert.

Die Kapsel 1 kann in der Kapselaufnahme 103 durch eine in Figur 6 dargestellte verschwenkbare Platte 123 fixiert werden. Zum Einsetzen der Kapsel 1 in die Kapselaufnahme 103 kann die Platte 123 aus der in Figur 6 dargestellten Position nach unten verschwenkt werden und die Kapselaufnahme 103 geöffnet werden. Alternativ oder zusätzlich kann die Platte 123 auch zum Öffnen der Kapselaufnahme 103 verschieblich sein. Die Verschränkung und/ oder Verschiebung kann manuell und/oder durch einen Schwenkantrieb 124 erfolgen. Ist die Kapsel 1 in ihrer Endposition in die Kapselaufnahme 103 eingesetzt, kann die Platte 123 die Kapselaufnahme 103 verschließen und den Boden der Kapselaufnahme 103 bilden. Die Heizplatte 105 kann derart an der Platte 123 angeordnet sein, dass bei geschlossener Kapselaufnahme 103 die Heizplatte 105 den Boden 4 der Kapsel 1 kontaktiert, vergleiche z.B. Figur 6.

Die Kühlvorrichtung 112 kann wie z.B. in Figur 6 gezeigt einen Lüfter, Gebläse oder dergleichen aufweisen. Der Verdampfer 100 kann einen Speicher 113, einen Zerstäuber 114 und ein Mundstück 101 aufweisen. In dem Speicher 113 kann bereits verdampfter Wirkstoff 6 und/oder verdampfter Hilfsstoff 10 vorgehalten werden. Damit kann auch beim "Kaltstart" des Verdampfers verdampfter Wirkstoff 6 und/oder Hilfsstoff 10 bereitgestellt werden. Zudem kann beispielsweise vorgesehen sein, dass durch einen vor der in der Verbindungsleitung zwischen erster Kapselaufnahme 104 und Speicher 113 angeordneten Klappe 111 ein Sensor zur Konzentrationsmessung des verdampften Wirkstoffs 6 und/oder Hilfsstoffs 10 angeordnet ist und die Klappe 111 nur dann durch die Steuer-und/oder Regelungseinheit 116 geöffnet wird oder ist, wenn eine vorgegebene Konzentration überschritten ist oder wird. Damit kann sichergestellt werden, dass am Mundstück 101 stets eine Mindestkonzentration an Wirkstoff 6 und/oder Hilfsstoff 110 zur Verfügung steht. Der Speicher 113 kann dabei dazu vorgesehen sein, den Wirkstoff 6 und/oder Hilfsstoff 10 mit vorgegebener Konzentration zu puffern, d. h. z.B. Versorgungslücken zu geringerer Konzentration, d. h. z.B. bei geschlossener Klappe 111, zu überbrücken. Der Speicher 113 kann auch dazu genutzt werden, die Konzentration des Wirkstoffs 6 und/oder des Hilfsstoffs 10 vor dem Mundstück zu homogenisieren. Der Speicher 113 kann ebenfalls beheizt und/oder gekühlt werden.

Zwischen Mundstück 101 und Speicher 113 ist bei der in Figur 6 gezeigten Ausführungsform ein Zerstäuber 114 angeordnet. Eine Klappe oder ein Ventil oder dergleichen kann zwischen Speicher 113 und Zerstäuber 114 angeordnet sein, so dass eine fluidische Verbindung zwischen Speicher 113 und Zerstäuber 114 durch die Klappe hergestellt oder getrennt werden kann. Durch den Zerstäuber 114 kann der verdampfte Wirkstoff 6 und/oder Hilfsstoff 10 in kleinere Tröpfchen zerstäubt werden. Die zerstreuten Tröpfchen können dabei eine derartige Tröpfchenverteilung aufweisen, dass sie z.B. gut in die Lunge des am Mundstück 101 ziehenden bzw. saugenden Benutzers dringen können bzw. gut lungengängig sind, wodurch sich eine bessere Aufnahme des Wirkstoffs 6 und/oder des Hilfsstoffs 10 durch den Benutzer ergeben kann. Der Verdampfer 100 kann zudem einen oder mehrere Filter 115 aufweisen.

Durch den Zerstäuber kann der verdampfte Wirkstoff und/oder Hilfsstoff zu intrathorakalen (lungengängigen) Aerosolpartikeln zerstäubt werden, wobei die Teilchen bevorzugt einen Durchmesser zwischen etwa 0,5 und 5,5 µm aufweisen können.

Durch Ziehen am Mundstück 101 und/oder einen Überdruck im Speicher 113 kann eine kleine konzentrierte Menge Wirkstoff 6 und/oder Hilfsstoff 10 in den Zerstäuber 114 bzw. das Saugrohr des Zerstäubers 114 angesaugt bzw. eingeführt werden. Der Zerstäuber 114 bzw. das Saugrohr kann eine Venturi-Düse und/oder Luftkanäle aufweisen, so dass durch den Venturi-Effekt beim Ansaugen das konzentrierte Aerosol entsprechend verdünnt und/oder zerstäubt wird. Damit kann der Anteil der lungengängigen Aerosolfraktion ganz erheblich gesteigert werden. Um große Teilchen zu filtern ist es denkbar, die angesaugte Luft in Rotation zu versetzen, sodass sich die großen Teilchen an der porösen Wand des Saugrohres niederschlagen bzw. dort ausgefiltert und abgeschieden werden. Im Saugrohr können auch entsprechende Filter angebracht sein, die durch ihre Dichte auch die Durchflussrate des Wirkstoffes 6 und/oder des Hilfsstoffs 10 steuern. Der Aerosolstrom in dem Saugrohr kann über eine Art Labyrinth bzw. einen labyrinthartigen Kanal 118 mit mindestens einer zusätzlichen Abrisskante herumgeleitet werden, wobei zu große Tröpfchen nicht nur infolge der Schwerkraft, sondern auch durch ihren Kontakt an einer derartigen Schikane infolge der Umleitung abgeschieden werden.

Der Zerstäuber 114 kann auch ein Düsenvernebler, ein Ultraschallvernebler oder ein Membranvernebler sein oder aufweisen. Bei Düsenverneblern erzeugt ein starker Luftstrom an einer Düse einen Unterdruck und zieht so Tröpfchen aus einem kapillaren System. Da die Tröpfchen unterschiedliche Größen aufweisen, hält eine Prallplatte zu große Tröpfchen zurück. Bei Ultraschallverneblern wird mittels eines vibrierenden Piezokristalls Ultraschall generiert, der für die Entstehung der Tropfen verantwortlich ist. Je höher die Frequenz gewählt wird, desto feiner werden die Tröpfchen. Die Frequenz beispielsweise 1-3 MHz betragen. Membranvernebler zeichnen sich durch eine sehr dünne, aus einer Vielzahl Mikrobohrungen bestehende Membran aus, die im kHz-Bereich schwingt. Durch diese Schwingungen fungiert jede Mikrobohrung als kleine Pumpe und produziert feinste Tröpfchen.

Der Verdampfer 100 kann einen nicht gezeigten Akkumulator aufweisen, der die Steuer-und/oder Regelungseinheit 116 und sämtliche andere in dem Verdampfer 100 aufgenommenen, strombenötigenden Komponenten mit elektrischem Strom versorgt. Der Verdampfer 100 kann an einer Außenseite einen Anschluss für ein Stromkabel zum Aufladen des Akkumulators aufweisen. Alternativ oder zusätzlich kann der Akkumulator austauschbar sein. Alternativ oder zusätzlich können auch nicht aufladbare Batterien vorgesehen sein.

Der Verdampfer 100 kann zumindest teilweise aus einem durchsichtigen Kunststoff bestehen oder einen solchen aufweisen. Bevorzugt kann der Verdampfer 100 zumindest im Bereich der Kapselaufnahme 103 und/oder die Kapselaufnahme 103 selbst aus einem durchsichtigen Kunststoff bestehen oder einen solchen aufweisen, oder zumindest durchsichtig sein, so dass eine in die Kapselaufnahme 103 eingesetzte Kapsel 1 von außenhalb des Verdampfers 100 sichtbar ist.

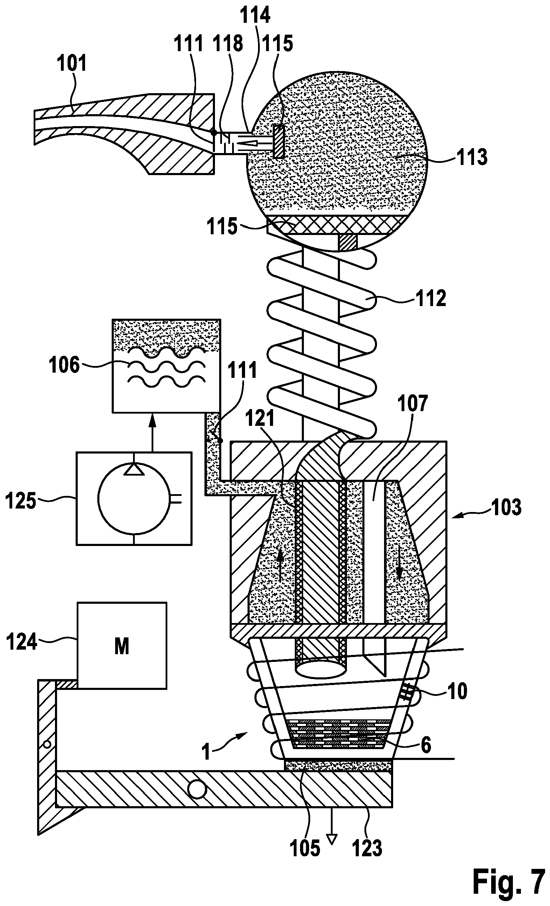

Figur 7 zeigt eine weitere Ausführungsform eines erfindungsgemäßen Verdampfers 100 mit einer in der Kapselaufnahme 103 aufgenommenen erfindungsgemäßen Kapsel 1. Die Verdampfungseinheit 102 weist ein Reservoir 106 mit einem chemischen Reaktionsmittel auf, wobei eine Pumpe 125 das Eindringen des chemischen Reaktionsmittel in Kapselaufnahme 103 unterstützt. Wie aus Figur 7 ersichtlich, kann der Verdampfer derart eingerichtet sein, dass das chemische Reaktionsmittel nicht in das Innere der Kapsel 1 eingeführt wird, sondern außerhalb der Kapsel 1 mit dem komplementären chemischen Mittel reagiert, welches beispielsweise als Beschichtung der Kapselaufnahme 103 und/oder der Außenseite des Deckels 2 der Kapsel vorliegt. Bei der in Figur 7 gezeigten Ausführungsform dient die zweite Kapselöffnungsvorrichtung 107 nicht der fluidischen Verbindung, sondern als Wärmeleiter zum Transport der durch die chemische Reaktion erzeugten Wärme und kann z.B. als metallischer Vollkörper oder als Heatpipe ausgeführt sein. Die zweite Kapselöffnungsvorrichtung 107 kann derart dimensioniert oder geformt sein, dass bei eingesetzter Kapsel die zweite Kapselöffnungsvorrichtung 107 den Wirkstoff 6 und/oder den Hilfsstoff 10 bevorzugt mit ihrer Spitze oder einem Endabschnitt kontaktiert oder in diesen eindringt. Die zweite Kapselöffnungsvorrichtung 107 kann auch derart geformt oder dimensioniert sein, dass beim Einsetzen der Kapsel durch die zweite Kapselöffnungsvorrichtung 107 die Innenwand 107 derart perforiert und/oder geöffnet wird, dass eine fluidische Verbindung zwischen erstem Hohlraum 5 und zweitem Hohlraum 10 geschaffen wird. Es kann aber alternativ oder zusätzlich auch vorgesehen sein, dass das chemische Reaktionsmittel in einer nicht in Figur 7 gezeigten Heizschlange in der Kapselaufnahme 103 geführt wird, wobei die Heizschlange mit einer Außenseite einer in die Kapselaufname 103 eingesetzten Kapsel 1 in Kontakt stehen kann. Die Heizschlange kann rohrförmig und gewunden sein. Die Heizschlange kann an ihrer Innenseite mit einem komplementären chemischen Mittel beschichtet sein, so dass das durch die Heizschlange geführte chemische Reaktionsmittel exothermisch reagieren kann. Die Verdampfereinheit 102 weist zudem eine mit Bezug auf Figur 6 beschriebene Heizplatte 105 auf. Weiterhin weist die Verdampfereinheit 102 eine in der Kapselaufnahme 103 angeordnete Heizspirale 109 auf, die von einem elektrischen Strom durchströmt und erwärmt werden kann. Durch die Heizspirale 109 kann der in der Kapsel aufgenommene Wirkstoff 6 und/oder der Hilfsstoff 10 erwärmt und verdampft werden.

Die Verdampfereinheit 102 kann alternativ oder zusätzlich einen nicht in der Figur 7 gezeigten Mikrowellengenerator aufweisen. Vorteilhafterweise ist in diesem Falle der in der Kapsel aufgenommene Wirkstoff 6 und oder Hilfsstoff 10 angefeuchtet, so dass der Wirkstoff 6 und/oder der Hilfsstoff 10 durch die von der Mikrowellengenerator erzeugten Mikrowellen erwärmt und verdampft werden kann.

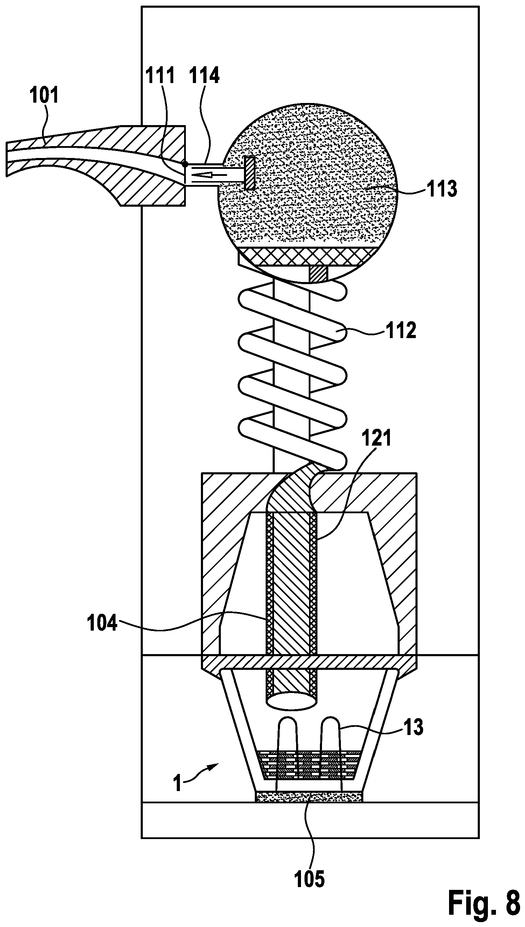

Figur 8 zeigt eine weitere Ausführungsform eines erfindungsgemäßen Verdampfers 100 mit einer in die Kapselaufnahme 103 eingesetzten erfindungsgemäßen Kapsel 1. Die Verdampfereinheit 102 weist dabei eine oben beschriebene Heizplatte 105 auf. Weiterhin ist im ersten Hohlraum 5 der Kapsel 1 eine Heizspule 13 angeordnet, die wie mit Bezug auf Figur 3 beschrieben durch einen elektrischen Strom erwärmt werden kann. Damit kann der Wirkstoff 6 und/oder der nicht in Figur 8 gezeigte Hilfsstoff 10 erwärmt und verdampft werden. Beispielsweise können die Kontakte der Heizspule 13 durch den Boden 4 der Kapsel einzeln durchgeführt werden und mit auf der Platte angeordneten entsprechenden Kontakten elektrisch leitend verbunden sein.

Das in Figur 8 gezeigte Ausführungsbeispiel weist nur eine erste Kapselöffnungsvorrichtung 104, nicht aber eine zweite Kapselöffnungsvorrichtung auf. In diesem Falle wird kein externes Fluid durch die Verdampfereinheit 102 der Kapsel 5 bereitgestellt. In einigen Ausführungsformen kann allerdings wie oben beschrieben eine zweite Kapselöffnungsvorrichtung vorgesehen sein, und/oder ein externes Fluid, beispielsweise erwärmte Luft aus einem Reservoir oder der Umgebung in die Kapsel geführt werden.

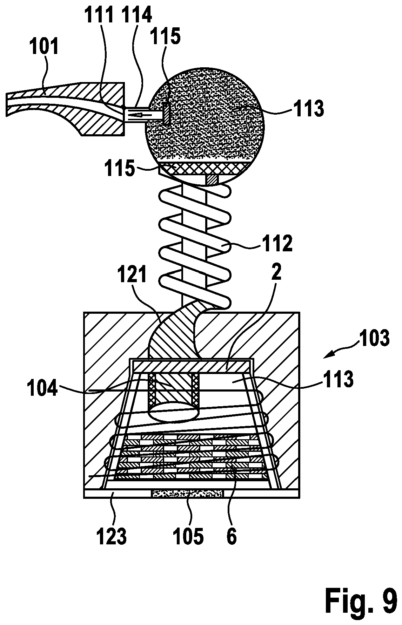

Figur 9 zeigt ein weiteres Ausführungsbeispiel eines erfindungsgemäßen Verdampfers 100 und einer in der Kapselaufnahme 103 aufgenommenen erfindungsgemäßen Kapsel 1. Die Verdampfereinheit 102 weist eine in der Kapselöffnung 103 angeordnete Heizspirale 109 zum Erwärmen des in der Kapsel aufgenommenen Wirkstoffs 6 und/oder des nicht in der Figur gezeigten Hilfsstoffs 10. Die Verdampfereinheit 102 weist zudem eine Heizplatte 105 auf. Die in Figur 9 gezeigte Ausführungsform weist keine zweite Kapselöffnungsvorrichtung auf. In einigen Ausführungsformen kann allerdings wie oben beschrieben eine zweite Kapselöffnungsvorrichtung vorgesehen sein, und/oder ein externes Fluid, beispielsweise erwärmte Luft aus einem Reservoir oder der Umgebung, in die Kapsel geführt werden.

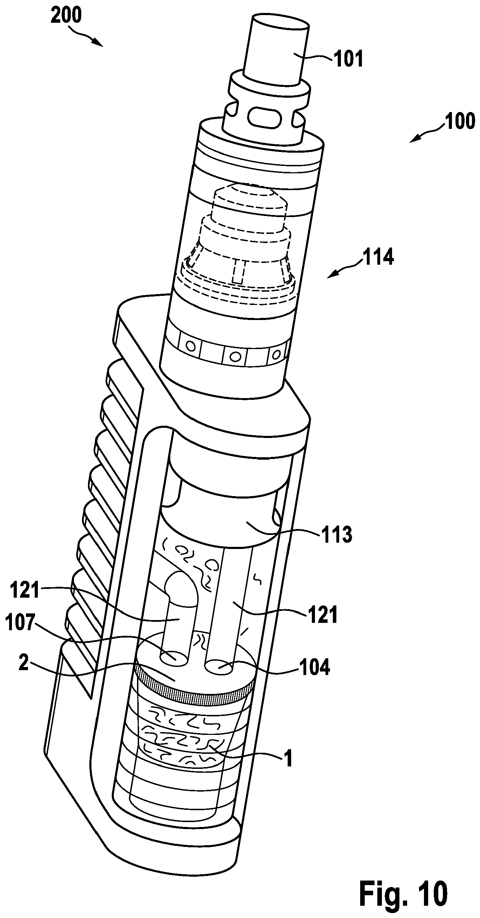

Figur 10 zeigt ein weiteres Ausführungsbeispiel eines erfindungsgemäßen Verdampfers 100 und einer in der Kapselaufnahme 103 aufgenommenen erfindungsgemäßen Kapsel 1 in einer perspektivischen Darstellung. Bei der gezeigten Ausführungsform wird Umgebungsluft in den Verdampfer 100 geführt, erwärmt und der Kapsel 1 über die zweite Kapselvorrichtung 107 zugeführt. Durch Wärmeübertragung der erwärmten Luft an den Wirkstoff 6 und/oder den Hilfsstoff 10 werden diese verdampft. Der verdampfte Wirkstoff 6 und/oder Hilfsstoff 10 wird durch die zweite Kapselöffnungsvorrichtung in dem Speicher 113 geleitet und dort gesammelt. Der verdampfte Wirkstoff 6 und/oder Hilfsstoff 10 wird von dem Speicher 113 durch den Zerstäuber 114 geführt, im Zerstäuber 114 zerstäubt und anschließend zum Mundstück 101 geführt.

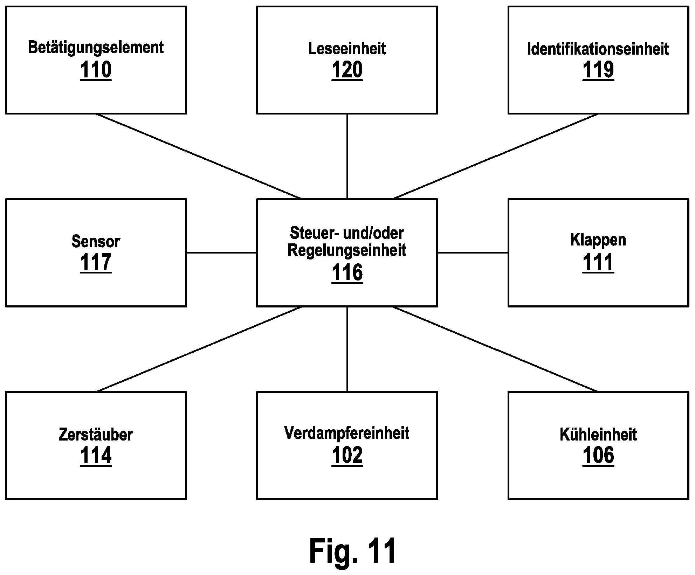

Figur 11 zeigt schematisch die Steuer-und/oder Regelungseinheit 116 sowie die damit zum Datenaustausch verbundenen Elemente bzw. Komponenten. Mit der Wortwahl "zum Datenaustausch verbundenen" kann ein Austausch von Informationen zwischen den jeweiligen Komponenten gemeint sein. Es kann aber auch gemeint sein, dass die Steuer-und/oder Regelungseinheit 116 eine jeweilige Komponente ansteuert bzw. Steuerungsbefehle übermittelt. Mindestens ein Sensor 117 ist mit der Steuer-und/oder Regelungseinheit 116 verbunden. Die Steuer-und/oder Regelungseinheit 116 kann ein Mikrocontroller, ein integrierter Schaltkreis, ein FPGA, und/oder ein Prozessor oder dergleichen sein oder aufweisen. Die Steuer-und oder Regelungseinheit 116 kann einen Datenspeicher aufweisen, in dem Informationen z.B. bezüglich des Verdampfertyps, des individuellen Verdampfers und/oder eines individuellen Benutzers hinterlegt sein können.

Der mindestens eine Sensor 117 kann ein Temperatursensor, ein Drucksensor, ein Sensor zur Messung der Strömungsgeschwindigkeit und/oder ein Sensor zur Konzentrationsmessung beispielsweise des Wirkstoffs 6, sein oder aufweisen. Mindestens ein Sensor 117 kann jeweils in dem ersten und/oder zweiten Hohlraum 5, 9 der Kapsel 1, der ersten und/oder zweiten Kapselöffnungsvorrichtung 104, 107, der Kapselaufnahme 103, dem Reservoir 106, den jeweiligen Verbindungsleitungen 121, der Kühleinheit 112, dem Speicher 113, dem Zerstäuber 114 und/oder dem Mundstück 101 angeordnet sein. Die von dem mindestens einen Sensor 117 gemessenen Messwerte können an die Steuer-und/oder Regelungseinheit 116 übermittelt und von dieser ausgewertet werden. Das Betätigungselement 110, die Leseeinheit 120 und/oder die Identifikationseinheit 119 können mit der Steuer-und/oder Regelungseinheit 116 verbunden sein und die erfassten Informationen, z.B. Betätigung des Betätigungselements, erfasster Code 14 oder Benutzeridentifikationsinformationen können an die Steuer-und/oder Regelungseinheit 116 übermittelt und von dieser ausgewertet werden. Bevorzugt kann die Identifikationseinheit 119 einen Fingerabdrucksensor 119 sein oder aufweisen. Die Steuer-und/oder Regelungseinheit 116 kann die Verdampfereinheit 102, die Kühleinheit 106, die Klappen 111 und/oder den Zerstäuber 114 ansteuern.

Die Ansteuerung kann von der Steuer-und/oder Regelungseinheit 116 durchgeführten Auswertung der Messwerte und/oder übermittelten Informationen und/oder der in den Datenspeicher hinterlegten Informationen abhängen. Enthält der Code 14 z.B. Informationen bezüglich des Wirkstoffs 6, so kann vorgesehen sein, dass die Steuer-und/oder Regelungseinheit 116 aus einer in ihrem Datenspeicher hinterlegten Datenbank eine Verdampfungstemperatur bestimmt und die Verdampfungseinheit 102 und/oder die Kühleinheit 112 derart ansteuert, dass die Verdampfungstemperatur erreicht und/oder eingehalten und/oder nicht überschritten wird. Gleichermaßen kann z.B. die Verdampfungstemperatur auch direkt in dem Code 14 enthalten sein und von der Steuer-und/oder Regelungseinheit 116 ausgewertet werden. In einigen Ausführungsformen kann beispielsweise alternativ oder zusätzlich in dem Code 14 enthaltene Informationen bezüglich des Wirkstoffs 6 und/oder das Hilfsstoffs 10 mit dem in dem Datenspeicher hinterlegten Informationen des individuellen Benutzers verglichen werden, um eine Berechtigung des Benutzers zum Verdampfen des Wirkstoffs 6 und/oder das Hilfsstoffs 10 zu ermitteln. Ist der Benutzer nicht berechtigt, so kann vorgesehen sein, dass die Steuer-und/oder Regelungseinheit 116 die Verdampfereinheit 102 und/oder die Klappen 111 nicht ansteuert, so dass der Wirkstoff 6 und/oder der Hilfsstoff 10 nicht verdampft wird. Ist oder enthält der Wirkstoff 6 beispielsweise ein zu verdampfendes Medikament, kann sichergestellt werden, dass der Benutzer kein falsches bzw. nicht verschriebenes Medikament durch den Verdampfer 100 einnimmt und/oder dass die Dosierung des Medikaments der verschriebenen entspricht. Gleichermaßen können die von dem Fingerabdrucksensor 119 erfassten Fingerabdrücke mit in den Datenspeicher hinterlegten Informationen bezüglich des individuellen Benutzers verglichen werden, so dass der Verdampfer beispielsweise nur von einem oder ggf. mehreren bestimmten Benutzern benutzt werden kann. Damit kann sich insbesondere eine bessere Hygiene ergeben. Es kann vorgesehen sein, dass der Verdampfer 100 an einer Außenseite ein Signalelement, beispielsweise eine LED, aufweist, das eine unberechtigte Benutzung bzw. nicht-Verdampfung des Wirkstoffs 6 und/oder das Hilfsstoffs 10 signalisiert. In einigen Ausführungsformen kann die LED auch dazu eingerichtet sein, beispielsweise einen Füllstand eines Akkumulators des Verdampfers 100 oder dergleichen anzuzeigen.

Die in den Ansprüchen, der Beschreibung, den Figuren und der Zusammenfassung offenbarten Merkmale können einzeln oder in Kombination für die Erfindung wesentlich sein.

**Bezugszeichenliste**

| | |
|---|---|
| Kapsel | 1 |
| Deckel | 2 |
| Seitenwand | 3 |
| Boden | 4 |
| erster Hohlraum | 5 |
| Wirkstoff | 6 |
| Innenwand | 7 |
| Außenwand | 8 |
| zweiter Hohlraum | 9 |
| Hilfsstoff | 10 |
| Beschichtung | 11 |
| Mahlkugel | 12 |
| Heizspule | 13 |
| Code | 14 |
| Verdampfer | 100 |
| Mundstück | 101 |
| Verdampfereinheit | 102 |
| Kapselaufnahme | 103 |
| erste Kapselöffnungsvorrichtung | 104 |
| Heizplatte | 105 |
| Reservoir | 106 |
| zweite Kapselöffnungsvorrichtung | 107 |
| Heizdraht | 108 |
| Heizspirale | 109 |
| Betätigungselement | 110 |
| Klappen | 111 |
| Kühleinheit | 112 |
| Speicher | 113 |
| Zerstäuber | 114 |
| Filter | 115 |
| Steuer- und/oder Regelungseinheit | 116 |
| Sensor | 117 |
| labyrinthartiger Kanal | 118 |
| Fingerabdrucksensor | 119 |
| Leseeinheit | 120 |
| Verbindungsleitung | 121 |
| Verdampfter Wirkstoff | 122 |
| Platte | 123 |
| Schwenkantrieb | 124 |
| Pumpe | 125 |
| Weitere Verbindungsleitung | 126 |
| Verdampfersystem | 200 |

## Patentansprüche

1. Kapsel (1) zur Verwendung in einem Verdampfer (100), mit mindestens einem zumindest teilweise von einem Deckel (2), Seitenwänden (3) und Boden (4) der Kapsel (1) gebildeten ersten Hohlraum (5) und einem in dem ersten Hohlraum (5) der Kapsel (1) aufgenommenen, verdampfbaren Wirkstoff (6), wobei die Seitenwände (3) und/oder der Boden (4) zumindest abschnittsweise doppelwandig mit einer inneren, dem ersten Hohlraum (5) der Kapsel zugewandten Innenwand (7) und einer der Außenseite der Kapsel zugewandten Außenwand (8) ausgebildet sind, so dass zwischen der Innenwand (7) und der Außenwand (8) mindestens ein zweiter Hohlraum (9) gebildet ist, wobei in dem zweiten Hohlraum (9) ein Hilfsstoff (10), bevorzugt ein weiterer Wirkstoff, ein chemisches Reaktionsmittel und/oder ein Geschmacksmittel aufgenommen ist, **dadurch gekennzeichnet, dass** der Boden (4) der Kapsel (1) zumindest an seiner Außenseite zumindest teilweise ein Material, bevorzugt ein Metall, mit einer verglichen mit dem Material der Seitenwand höheren Wärmeleitfähigkeit aufweist.

2. Kapsel (1) nach Anspruch 1, bei die Außenwand (8), der Deckel (2) und der Boden (4) luftundurchlässig sind, so dass der erste und/oder der zweite Hohlraum (5, 9) hermetisch gegen eine Umgebung der Kapsel (1) abgeschlossen ist.

3. Kapsel (1) nach einem der vorangegangenen Ansprüche, bei der der erste und/ oder der zweite Hohlraum (5, 9) entweder evakuiert oder zumindest teilweise mit einem Schutzgas befüllt ist.

4. Kapsel (1) nach einem der vorangegangenen Ansprüche, bei der die zwischen dem ersten und zweiten Hohlraum (5, 9) angeordnete Innenwand (8) der Seitenwand (3) perforierbar, porös und/oder semi-permeabel ist und/oder Sollbruchstellen aufweist.

5. Kapsel (1) nach einem der vorangegangenen Ansprüche, bei der der Deckel (2) perforierbar ist und/oder Sollbruchstellen und/oder Ventile aufweist.

6. Kapsel (1) nach einem der vorangegangenen Ansprüche, bei der der Wirkstoff (6) und/oder der Hilfsstoff (10) eine Cannabisblüte, ein Cannabisöl, ein Cannabinoid, ein Cannabisextrakt oder dergleichen ist oder aufweist.

7. Kapsel (1) nach einem der vorangegangenen Ansprüche, bei der der Deckel (2) und/oder der Boden (4) an einer dem Hohlraum (5) zugewandten Innenseite und/oder die Innenwand (7) mindestens teilweise eine Beschichtung (11) aufweist, wobei die Beschichtung (11) bevorzugt ein chemisches Reaktionsmittel, einen UV-Filter und/oder ein thermochromes Material aufweist.

8. Kapsel (1) nach einem der vorangegangenen Ansprüche, bei der in dem ersten Hohlraum (5) eine oder mehrere Mahlkugeln (12) zum Zerkleinern des Wirkstoffs (6) aufgenommen sind, wobei die Mahlkugeln (12) ein ferromagnetisches Material aufweisen.

9. Kapsel (1) nach einem der vorangegangenen Ansprüche, bei der die Innenwand (7) und/oder die Außenwand (8) zumindest abschnittsweise aus einem Glas und/oder einem durchsichtigen Kunststoff besteht oder aufweist.

10. Kapsel (1) nach einem der vorangegangenen Ansprüche, bei der in dem ersten Hohlraum (5) und/oder dem zweiten Hohlraum (9) eine Heizspule (13) angeordnet ist.

11. Kapsel (1) nach einem der vorangegangenen Ansprüche, bei der auf der Außenseite des Deckels (2) und/oder des Bodens (4) der Kapsel (1) einen Code (14), bevorzugt einen QR-Code, einen alphanumerischen Code, einen Barcode und/oder ein Piktogramm, und/oder einen RFID-Chip aufweist.

12. Anordnung aus einer Kapsel (1) nach einem der vorangegangenen Ansprüche und einem Verdampfer (100) zum Verdampfen von Wirkstoffen (6, 10), mit einem Mundstück (101) und einer Verdampfereinheit (102), wobei der Verdampfer (100) eine Kapselaufnahme (103) zur Aufnahme der Kapsel (1) aufweist, wobei die Kapselaufnahme (103) eine erste Kapselöffnungsvorrichtung (104) aufweist und über die erste Kapselöffnungsvorrichtung (104) mit dem Mundstück (101) fluidisch verbunden ist, so dass bei aufgenommener Kapsel (1) eine fluidische Verbindung zwischen einem Hohlraum (5, 9) der Kapsel und dem Mundstück (101) durch die erste Kapselöffnungsvorrichtung (104) bereitgestellt werden kann, wobei die Verdampfereinheit (102) zum Verdampfen eines sich bei aufgenommener Kapsel (1) in dem Hohlraum (5, 10) der Kapsel (1) befindlichen Wirkstoffs (6, 10) eingerichtet ist.

13. Anordnung nach Anspruch 12, bei dem die Verdampfereinheit (102) eine Heizplatte (105) in der Kapselaufnahme (103) ist oder aufweist, so dass bei aufgenommener Kapsel (1) die Heizplatte (105) eine Außenseite, bevorzugt einen Boden (4) der Kapsel (1), kontaktiert.

14. Anordnung nach einem der vorangegangenen Ansprüche 12 und 13, bei dem die Verdampfereinheit (102) ein Reservoir (106) zur Aufnahme eines Fluids ist oder aufweist, wobei das Reservoir (106) über eine zweite Kapselöffnungsvorrichtung (107) mit der Kapselaufnahme (103) fluidisch verbunden ist, so dass bei aufgenommener Kapsel (1) eine fluidische Verbindung zwischen einem Hohlraum (5, 9) der Kapsel (1) und dem Reservoir (106) durch die zweite Kapselöffnungsvorrichtung (107) bereitgestellt werden kann und
a. einen oder mehrere Heizdrähte (108) in dem Reservoir (106) und/oder fluidisch zwischen Reservoir (106) und Kapselaufnahme (103) zur Erwärmung des Fluids aufweist und/oder
b. das Fluid in dem Reservoir (106) aufgenommen ist, wobei das Fluid ein chemisches Reaktionsmittel ist oder aufweist, so dass durch eine chemische Reaktion des chemischen Reaktionsmittels mit einem komplementären chemischen Mittel Wärme freigesetzt wird.

15. Anordnung nach einem der vorangegangenen Ansprüche 12 bis 14, bei dem die Verdampfereinheit (102) ein Mikrowellengenerator ist oder aufweist.

16. Anordnung nach einem der vorangegangenen Ansprüche 12 bis 15, bei dem die Verdampfereinheit (102) eine Heizspirale(109) in der Kapselaufnahme (103) ist oder aufweist.

17. Anordnung nach einem der vorangegangenen Ansprüche 12 bis 15, bei dem der Verdampfer (100) ein Betätigungselement (110) aufweist, wobei die erste und/oder mit Bezug auf Anspruch 12 b) ggf. die zweite Kapselöffnungsvorrichtung (104, 107)
a. Klappen (111) aufweisen, die bei Betätigen des Betätigungselements (110) geöffnet werden und/oder
b. bei Betätigen des Betätigungselements (110) in Richtung Kapselaufnahme (103) verfahren werden.

18. Anordnung nach einem der vorangegangenen Ansprüche 12 bis 17, bei dem der Verdampfer (100) eine fluidisch zwischen Kapselaufnahme (103) und Mundstück (101) angeordnete Kühleinheit (112) aufweist, so dass von dem Verdampfer (100) verdampfter Wirkstoff (6, 10) gekühlt wird, bevorzugt auf eine Temperatur von 10 - 30°C, besonders bevorzugt auf eine Temperatur von 15 - 25°C.

19. Anordnung nach einem der Ansprüche 12 bis 18, bei dem der Verdampfer (100) einen dem Mundstück (101) fluidisch vorgelagerten Speicher (113) aufweist, in welchem von dem Verdampfer (100) verdampfter Wirkstoff (6, 10) gesammelt werden kann.

20. Anordnung nach einem der Ansprüche 12 bis 19, bei dem das Mundstück (101) einen Zerstäuber (114) zum Zerstäuben von verdampftem Wirkstoff (6, 10) in Tröpfchen aufweist.

21. Anordnung nach einem der Ansprüche 12 bis 20, bei dem das Mundstück (101), der Zerstäuber (114) und/oder die erste und/oder bei Bezug auf Anspruch 12 b) die zweite Kapselöffnungsvorrichtung (104, 107) eine Venturi-Düse ist oder aufweist und/oder einen hyperbolischen Querschnitt in Strömungsrichtung aufweist.

22. Anordnung nach einem der Ansprüche 12 bis 21, bei dem der Verdampfer (100) mindestens einen dem Mundstück (101) fluidisch vorgelagerten Filter (115) aufweist, wobei der Filter (115) bevorzugt an dem Mundstück (101) angeordnet ist.

23. Anordnung nach einem der Ansprüche 12 bis 23, bei dem der Verdampfer (100) eine Steuer- und/oder Regelungseinheit (116) und mindestens einen Sensor (117), bevorzugt einen Temperatursensor, zur Steuerung und Regelung der Temperatur der Verdampfereinheit (102) aufweist.

24. Anordnung nach einem der Ansprüche 12 bis 23, bei dem der Zerstäuber (114) einen labyrinthartigen Kanal (118) mit mindestens einer Abrisskante aufweist, so dass durch den Kanal strömende Tröpfchen größer als eine maximale Tröpfchengröße abgeschieden werden können.

25. Anordnung nach einem der Ansprüche 12 bis 24, der einen Fingerabdrucksensor (119) aufweist, wobei der Fingerabdrucksensor (119) bevorzugt derart angeordnet ist, dass bei einhändiger Benutzung des Verdampfers (100), besonders bevorzugt bei Bezug auf Anspruch x bei einhändiger Betätigung des Betätigungselements (110), der Fingerabdrucksensor (119) einen Fingerabdruck erfasst.

26. Anordnung nach einem der Ansprüche 12 bis 25, der eine Leseeinheit (120) aufweist, wobei die Leseeinheit (120) dazu eingerichtet ist, bei eingesetzter Kapsel (1) einen auf einer Außenseite (2) der Kapsel (1) angebrachten Code (121), bevorzugt einen QR-Code, einen alphanumerischen Code, einen Barcode und/oder ein Piktogramm, und/oder RFID-chip zu erfassen und/oder auszulesen, wobei die Leseeinheit bevorzugt in der Kapselaufnahme (103) angeordnet ist.

27. Verdampfersystem (200) zum Verdampfen eines Wirkstoffs (6, 10), aufweisend eine Anordnung nach einem der Ansprüche 12 bis 27 und eine in der Kapselaufnahme (103) des Verdampfers (100) aufgenommene Kapsel (1) nach einem der Ansprüche 1 bis 11.

## Claims

1. A capsule (1) for use in an evaporator (100), having at least one first cavity (5), which is formed at least partially by a cover (2), side walls (3) and base (4) of the capsule (1), and an active substance (6) which can be evaporated and is accommodated in the first cavity (5) of the capsule (1), wherein the side walls (3) and/or the base (4) are of double-walled design, at least in sections, with an inner wall (7), which faces the first cavity (5) of the capsule, and an outer wall (8), which faces the outer side of the capsule, such that at least one second cavity (9) is formed between the inner wall (7) and the outer wall (8), wherein an auxiliary substance (10), preferably a further active substance, a chemical reactant and/or a flavoring agent is accommodated in the second cavity (9), **characterized in that** the base (4) of the capsule (1) has, at least on its outer side, at least partially a material, preferably a metal, with a higher thermal conductivity compared with the material of the side wall.

2. The capsule (1) according to claim 1, wherein the outer wall (8), the cover (2) and the base (4) are impermeable to air, such that the first and/or the second cavity (5, 9) is hermetically sealed against an environment of the capsule (1).

3. The capsule (1) according to one of the preceding claims, wherein the first and/or the second cavity (5, 9) is either evacuated or at least partially filled with a protective gas.

4. The capsule (1) according to one of the preceding claims, wherein the inner wall (8) of the side wall (3), which is arranged between the first and second cavity (5, 9), can be perforated, is porous and/or semi-permeable and/or has predetermined breaking points.

5. The capsule (1) according to one of the preceding claims, wherein the cover (2) can be perforated and/or has predetermined breaking points and/or valves.

6. The capsule (1) according to one of the preceding claims, wherein the active substance (6) and/or the auxiliary substance (10) is or has a cannabis flower, a cannabis oil, a cannabinoid, a cannabis extract or the like.

7. The capsule (1) according to one of the preceding claims, wherein the cover (2) and/or the base (4) has, on an inner side facing the cavity (5) and/or the inner wall (7), at least partially a coating (11), wherein the coating (11) preferably has a chemical reactant, a UV filter and/or a thermochromic material.

8. The capsule (1) according to one of the preceding claims, wherein one or more grinding balls (12) for comminuting the active substance (6) are accommodated in the first cavity (5), wherein the grinding balls (12) have a ferromagnetic material.

9. The capsule (1) according to one of the preceding claims, wherein the inner wall (7) and/or the outer wall (8) consists or has, at least in sections, a glass and/or a transparent plastic.

10. The capsule (1) according to one of the preceding claims, wherein a heating coil (13) is arranged in the first cavity (5) and/or the second cavity (9).

11. The capsule (1) according to one of the preceding claims, wherein a code (14), preferably a QR code, an alphanumeric code, a barcode and/or a pictogram, and/or an RFID chip is provided on the outer side of the cover (2) and/or the base (4) of the capsule (1).

12. An arrangement consisting of a capsule (1) according to one of the preceding claims and an evaporator (100) for evaporating active substances (6, 10), having a mouthpiece (101) and an evaporator unit (102), wherein the evaporator (100) has a capsule receptacle (103) for receiving the capsule (1), wherein the capsule receptacle (103) has a first capsule opening device (104) and is fluidically connected to the mouthpiece (101) via the first capsule opening device (104), so that, when the capsule (1) is received, a fluidic connection between a cavity (5, 9) of the capsule and the mouthpiece (101) can be provided by the first capsule opening device (104), wherein the evaporator unit (102) is configured for evaporating an active substance (6, 10) located in the cavity (5, 10) of the capsule (1) when the capsule (1) is received.

13. The arrangement according to claim 12, wherein the evaporator unit (102) is or has a heating plate (105) in the capsule receptacle (103), so that, when the capsule (1) is received, the heating plate (105) contacts an outer side, preferably a base (4) of the capsule (1).

14. The arrangement according to one of the preceding claims 12 and 13, wherein the evaporator unit (102) is or has a reservoir (106) for receiving a fluid, wherein the reservoir (106) is fluidically connected to the capsule receptacle (103) via a second capsule opening device (107), so that, when the capsule (1) is received, a fluidic connection between a cavity (5, 9) of the capsule (1) and the reservoir (106) can be provided by the second capsule opening device (107), and
a. one or more heating wires (108) in the reservoir (106) and/or fluidically between the reservoir (106) and the capsule receptacle (103) for heating the fluid, and/or
b. the fluid is received in the reservoir (106), wherein the fluid is or has a chemical reactant, so that heat is released by a chemical reaction of the chemical reactant with a complementary chemical agent.

15. The arrangement according to one of the preceding claims 12 to 14, wherein the evaporator unit (102) is or has a microwave generator.

16. The arrangement according to one of the preceding claims 12 to 15, wherein the evaporator unit (102) is or has a heating coil (109) in the capsule receptacle (103).

17. The arrangement according to one of the preceding claims 12 to 15, wherein the evaporator (100) has an actuating element (110), wherein the first and/or with reference to claim 12 b) optionally the second capsule opening device (104, 107)
a. have flaps (111) which are opened when the actuating element (110) is actuated, and/or
b. are moved in the direction of the capsule receptacle (103) when the actuating element (110) is actuated.

18. The arrangement according to one of the preceding claims 12 to 17, wherein the evaporator (100) has a cooling unit (112) arranged fluidically between the capsule receptacle (103) and the mouthpiece (101), so that active substance (6, 10) evaporated by the evaporator (100) is cooled, preferably to a temperature of 10-30°C, particularly preferably to a temperature of 15-25°C.

19. The arrangement according to one of claims 12 to 18, wherein the evaporator (100) has a reservoir (113) which is arranged fluidically upstream of the mouthpiece (101) and in which active substance (6, 10) evaporated by the evaporator (100) can be collected.

20. The arrangement according to one of claims 12 to 19, wherein the mouthpiece (101) has an atomizer (114) for atomizing evaporated active substance (6, 10) into droplets.

21. The arrangement according to one of claims 12 to 20, wherein the mouthpiece (101), the atomizer (114) and/or the first and/or with reference to claim 12 b) the second capsule opening device (104, 107) is or has a Venturi nozzle and/or has a hyperbolic cross section in the flow direction.

22. The arrangement according to one of claims 12 to 21, wherein the evaporator (100) has at least one filter (115) arranged fluidically upstream of the mouthpiece (101), wherein the filter (115) is preferably arranged on the mouthpiece (101).

23. The arrangement according to one of claims 12 to 23, wherein the evaporator (100) has a control and/or regulating unit (116) and at least one sensor (117), preferably a temperature sensor, for controlling and regulating the temperature of the evaporator unit (102).

24. The arrangement according to one of claims 12 to 23, wherein the atomizer (114) has a labyrinth-like channel (118) with at least one tear-off edge, so that droplets flowing through the channel can be deposited larger than a maximum droplet size.

25. The arrangement according to one of claims 12 to 24, which has a fingerprint sensor (119), wherein the fingerprint sensor (119) is preferably arranged in such a way that when the evaporator (100) is used with one hand, particularly preferably with reference to claim x when the actuating element (110) is actuated with one hand, the fingerprint sensor (119) detects a fingerprint.

26. The arrangement according to one of claims 12 to 25, which has a reading unit (120), wherein the reading unit (120) is configured to detect and/or read out a code (121), preferably a QR code, an alphanumeric code, a barcode and/or a pictogram, and/or an RFID chip attached to an outer side (2) of the capsule (1) when the capsule (1) is inserted, wherein the reading unit is preferably arranged in the capsule receptacle (103).

27. An evaporator system (200) for evaporating an active substance (6, 10), having an arrangement according to one of claims 12 to 27 and a capsule (1) according to one of claims 1 to 11 received in the capsule receptacle (103) of the evaporator (100).

## Revendications

1. Capsule (1) destinée à être utilisée dans un évaporateur (100), avec au moins une première cavité (5) constituée au moins partiellement d'un couvercle (2), de parois latérales (3) et d'un fond (4) de la capsule (1), et un principe actif (6) évaporable logé dans la première cavité (5) de la capsule (1), dans laquelle les parois latérales (3) et/ou le fond (4) sont réalisés, au moins à certains endroits, avec des doubles parois, avec une paroi intérieure (7) orientée vers la première cavité (5) de la capsule et une paroi extérieure (8) orientée vers l'extérieur de la capsule, de sorte que, entre la paroi intérieure (7) et la paroi extérieure (8), est formée au moins une deuxième cavité (9), dans laquelle, dans la deuxième cavité (9), est logé un agent auxiliaire (10), de préférence un autre principe actif, un agent de réaction chimique et/ou un agent aromatisant, **caractérisée en ce que** le fond (4) de la capsule (1) comprend, au moins sur son côté externe, au moins partiellement, un matériau, de préférence un métal, avec une conductivité thermique plus élevée que celle du matériau de la paroi latérale.

2. Capsule (1) selon la revendication 1, dans laquelle la paroi extérieure (8), le couvercle (2) et le fond (4) sont imperméables à l'air, de sorte que la première et/ou la deuxième cavité (5, 9) est fermée hermétiquement vis-à-vis d'un environnement de la capsule (1).

3. Capsule (1) selon l'une des revendications précédentes, dans laquelle la première et/ou la deuxième cavité (5, 9) est soit évacuée soit remplie au moins partiellement d'un gaz protecteur.

4. Capsule (1) selon l'une des revendications précédentes, dans laquelle la paroi externe (8) de la paroi latérale (3), disposée entre les première et deuxième cavités (5, 9), est perforable, poreuse et/ou semi-perméable et/ou présente des points de rupture prédéfinis.

5. Capsule (1) selon l'une des revendications précédentes, dans laquelle le couvercle (2) est perforable et/ou présente des points de rupture prédéfinis et/ou des soupapes.

6. Capsule (1) selon l'une des revendications précédentes, dans laquelle le principe actif (6) et/ou l'agent auxiliaire (10) est ou comprend une fleur de cannabis, une huile de cannabis, un cannabinoïde, un extrait de cannabis ou similaire.

7. Capsule (1) selon l'une des revendications précédentes, dans laquelle le couvercle (2) et/ou le fond (4) comprend, sur un côté interne orienté vers la cavité (5) et/ou la paroi interne (7) comprend au moins partiellement un revêtement (11), dans laquelle le revêtement (11) comprend de préférence un agent de réaction chimique, un filtre à UV et/ou un matériau thermochromique.

8. Capsule (1) selon l'une des revendications précédentes, dans laquelle, dans la première cavité (5), sont logées une ou plusieurs billes de broyage (12) pour le broyage du principe actif (6), dans laquelle les billes de broyage (12) comprennent un matériau ferromagnétique.

9. Capsule (1) selon l'une des revendications précédentes, dans laquelle la paroi interne (7) et/ou la paroi externe (8) est constituée, au moins à certains endroits, un verre et/ou une matière plastique transparente.

10. Capsule (1) selon l'une des revendications précédentes, dans laquelle, dans la première cavité (5) et/ou la deuxième cavité (9), est disposée une bobine de chauffage (13).

11. Capsule (1) selon l'une des revendications précédentes, dans laquelle, sur le côté externe du couvercle (2) et/ou du fond (4), la capsule (1) présente un code (14), de préférence un QR code, un code alphanumérique, un code barre, et/ou un pictogramme, et/ou une puce RFID.

12. Disposition constituée d'une capsule (1) selon l'une des revendications précédentes et d'un évaporateur (100) pour l'évaporation de principes actifs (6, 10) avec un élément d'embouchure (101) et une unité d'évaporateur (102), dans laquelle l'évaporateur (100) comprend un logement de capsule (103) pour le logement de la capsule (1), dans laquelle le logement de capsule (103) comprend un premier dispositif d'ouverture de capsule (104) et par l'intermédiaire duquel le premier dispositif d'ouverture de capsule (104) est relié de manière fluidique avec l'élément d'embouchure (101), de sorte que, lorsque la capsule (1) est logée, une liaison fluidique entre une cavité (5, 9) de la capsule et l'élément d'embouchure (101) peut être mise à disposition par le premier dispositif d'ouverture de capsule (104), dans laquelle l'unité d'évaporateur (102) est conçue pour l'évaporation d'un principe actif (6, 10) se trouvant, lorsque la capsule (1) est logée, dans la cavité (5, 10) de la capsule (1).

13. Disposition selon la revendication 12, dans laquelle l'unité d'évaporateur (102) est ou comprend une plaque chauffante (105) dans le logement de capsule (103), de sorte que, lorsque la capsule (1) est logée, la plaque chauffante (105) entre en contact avec un côté externe, de préférence un fond (4) de la capsule (1).

14. Disposition selon l'une des revendications précédentes 12 et 13, dans laquelle l'unité d'évaporateur (102) est ou comprend un réservoir (106) pour le logement d'un fluide, dans laquelle le réservoir (106) est relié de manière fluidique par l'intermédiaire d'un deuxième dispositif d'ouverture de capsule (107) avec le logement de capsule (103), de sorte que, lorsque la capsule (1) est logée, une liaison fluidique entre une cavité (5, 9) de la capsule (1) et le réservoir (106) peut être mise à disposition par le deuxième dispositif d'ouverture de capsule (107) et
a. comprend un ou plusieurs fils chauffants (108) dans le réservoir (106) et/ou de manière fluidique entre le réservoir (106) et le logement de capsule (103) pour le chauffage du fluide et/ou
b. le fluide est logé dans le réservoir (106), dans laquelle le fluide est ou comprend un agent de réaction chimique, de sorte qu'une réaction chimique de l'agent de réaction chimique avec un agent chimique complémentaire dégage de la chaleur.

15. Disposition selon l'une des revendications précédentes 12 à 14, dans laquelle l'unité d'évaporateur (102) est ou comprend un générateur de micro-ondes.

16. Disposition selon l'une des revendications précédentes 12 à 15, dans laquelle l'unité d'évaporateur (102) est ou comprend une spirale chauffante (109) dans le logement de capsule (103).

17. Disposition selon l'une des revendications précédentes 12 à 15, dans laquelle l'unité d'évaporateur (102) comprend un élément d'actionnement (110), dans laquelle le premier et/ou, en référence à la revendication 12 b), le cas échéant, le deuxième dispositif d'ouverture de capsule (104, 107)
a. comprennent des clapets (111) qui sont ouverts lors de l'actionnement de l'élément d'actionnement (110) et/ou
b. sont déplacés en direction du logement de capsule (103) lors de l'actionnement de l'élément d'actionnement (110).

18. Disposition selon l'une des revendications précédentes 12 à 17, dans laquelle l'évaporateur (100) comprend une unité de refroidissement (112) disposée de manière fluidique entre le logement de capsule (103) et l'élément d'embouchure (101), de sorte que l'agent auxiliaire (6, 10) évaporé par l'évaporateur (100) est refroidi de préférence à une température de 10 - 30 °C, plus particulièrement de préférence à une température de 15 - 25 °C.

19. Disposition selon l'une des revendications 12 à 18, dans laquelle l'évaporateur (100) comprend un accumulateur (113), disposé de manière fluidique en amont de l'élément d'embouchure (101), dans lequel l'agent auxiliaire (6, 10) évaporé par l'évaporateur (100) peut être collecté.

20. Disposition selon l'une des revendications 12 à 19, dans laquelle l'élément d'embouchure (101) comprend un pulvérisateur (114) pour la pulvérisation de l'agent auxiliaire (6, 10) évaporé en gouttelettes.

21. Disposition selon l'une des revendications 12 à 20, dans laquelle l'élément d'embouchure (101), le pulvérisateur (114) et/ou le premier et/ou, en référence à la revendication 12 b), le cas échéant, le deuxième dispositif d'ouverture de capsule (104, 107), est ou comprend une buse Venturi et/ou présente une section transversale hyperbolique dans la direction de l'écoulement.

22. Disposition selon l'une des revendications 12 à 21, dans laquelle l'évaporateur (100) comprend au moins un filtre (115), disposé de manière fluidique en amont de l'élément d'embouchure (101), dans laquelle le filtre (115) est disposé de préférence sur l'élément d'embouchure (101).

23. Disposition selon l'une des revendications 12 à 23, dans laquelle l'évaporateur (100) comprend une unité de commande et/ou de régulation (116) et au moins un capteur (117), de préférence un capteur de température, pour le contrôle et la régulation de la température de l'unité d'évaporateur (102).

24. Disposition selon l'une des revendications 12 à 23, dans laquelle le pulvérisateur (114) comprend un canal labyrinthique (118) avec au moins une arête de contour, de sorte que les gouttelettes s'écoulant à travers le canal, plus grandes qu'une taille maximale de gouttelettes, peuvent être déposées.

25. Disposition selon l'une des revendications 12 à 24, qui comprend un capteur d'empreinte digitale (119), dans laquelle le capteur d'empreinte digitale (119) est disposé de préférence de sorte qu'une utilisation d'une seule main de l'évaporateur (100), plus particulièrement de préférence, en référence à la revendication x, lors de l'actionnement d'une seule main de l'élément d'actionnement (110), le capteur d'empreinte digitale (119) détecte une empreinte digitale.

26. Disposition selon l'une des revendications 12 à 25, qui comprend une unité de lecture (120), dans laquelle l'unité de lecture (120) est conçue pour détecter et/ou lire, lorsque la capsule (1) est insérée, un code (121) appliqué sur un côté externe (2) de la capsule (1), de préférence un QR code, un code alphanumérique, un code barre et/ou un pictogramme et/ou une puce RFID, dans laquelle l'unité de lecture est disposée de préférence dans le logement de capsule (103).

27. Système d'évaporateur (200) pour l'évaporation d'un principe actif (6, 10), comprenant une disposition selon l'une des revendications 12 à 27 et une capsule (1), logée dans le logement de capsule (103) de l'évaporateur (100), selon l'une des revendications 1 à 11.
